# EUROPÄISCHE PATENTSCHRIFT

(11) **EP 1 015 402 B1**
(45) Veröffentlichungstag und Bekanntmachung des Hinweises auf die Patenterteilung: **04.12.2002**
(21) Anmeldenummer: 98947443.2
(22) Anmeldetag: 20.08.1998
(51) Int. Cl.: C07B 39/00, C07C 45/63, C07C 253/30

(54) **Verfahren zur Herstellung von Fluor enthaltenden Verbindungen, insbesondere Fluorbenzaldehyden und Fluorbenzonitrilen**
Method for producing compounds containing fluorine, in particular fluorobenzaldehydes and fluorobenzonitriles
Procédé de production de composés contenant du fluor, en particulier de fluorobenzaldehydes et de fluorobenzonitriles

(30) Priorität: 02.09.1997 DE 19738196
(43) Veröffentlichungstag der Anmeldung: 05.07.2000
(73) Patentinhaber: Clariant GmbH, 65929 Frankfurt am Main (DE)
(72) Erfinder: APPEL, Wolfgang, D-65779 Kelkheim (DE); PASENOK, Sergej, D-65779 Kelkheim (DE); WESSEL, Thomas, D-60386 Frankfurt am Main (DE)
(74) Vertreter: Brundin, Eike
(86) Internationale Anmeldenummer: EP9805296
(87) Internationale Veröffentlichungsnummer: WO99011588

(56) Entgegenhaltungen:
- EP-A- 0 265 854
- EP-A- 0 635 486
- WO-A-98/05610
- WO-A-98/22413
- FR-A- 2 275 426
- PATENT ABSTRACTS OF JAPAN vol. 096, no. 008, 30. August 1996 & JP 08 092148 A (IHARA CHEM IND CO LTD), 9. April 1996 in der Anmeldung erwähnt
- DATABASE WPI Section Ch, Week 9335 Derwent Publications Ltd., London, GB; Class E14, AN 93-278233 XP002085380 & JP 05 194303 A (IHARA CHEM IND CO LTD) , 3. August 1993 in der Anmeldung erwähnt
- DATABASE WPI Section Ch, Week 8813 Derwent Publications Ltd., London, GB; Class E14, AN 88-088421 XP002085381 & JP 63 039824 A (SHIN-AKITA KASEI KK) , 20. Februar 1988 in der Anmeldung erwähnt

## Beschreibung

Die vorliegende Erfindung bezieht sich auf ein Verfahren zur Herstellung von Fluor enthaltenden Verbindungen, vorzugsweise Fluor enthaltenden aromatischen Verbindungen, insbesondere Fluorbenzaldehyden und Fluorbenzonitrilen in hoher Reinheit.

Insbesondere betrifft die Erfindung ein gegenüber dem Stand der Technik verbessertes Verfahren, bei dem die Fluorierung durch eine Halogen-Fluor-Austauschreaktion (Halex-Verfahren) in hoher Selektivität und Reinheit erzielt wird.

Fluor enthaltende Verbindungen finden unter anderem in flüssigkristallinen Mischungen Anwendung (EP-A-0 602 596).

Die Substitution von aromatisch gebundenem Wasserstoff durch Fluor hat auch große Bedeutung für die Synthese von bioaktiven Substanzen respektive für die Hersteilung von Vorstufen zu solchen Verbindungen.

Ferner ist es allgemein bekannt, daß Fluor starke und oftmals unerwartete Effekte auf die biologische Aktivität von chemischen Verbindungen hat. Der Austausch von Wasserstoff gegen Fluor in einem biologisch aktiven Molekül führt oft zu einer analogen Verbindung mit erhöhter oder modifizierter biologischer Wirkung.

Neben der Direktffuorierung ist die Herstellung von Fluorverbindungen durch Austausch eines Halogens (Cl, Br) gegen Fluor (sogenannter "Halexprozess") eine äußerst wertvolle Reaktion, die von großer industrieller Bedeutung ist.

Bei aromatischen Verbindungen, insbesondere bei aktivierten aromatischen Verbindungen, verläuft der Halogen-Fluor-Austausch im Sinne einer nucleophilen Substitution.

Zur Durchführung dieser Reaktion werden vergleichsweise hohe Reaktionstemperaturen benötigt, die häufig zwischen 200 und 300 °C liegen, wodurch zum Teil erhebliche Anteile an Zersetzungsprodukten entstehen. Im allgemeinen kann auf ein Lösungsmittel nicht verzichtet werden, so daß die Raum/Zeit-Ausbeuten im Vergleich zu lösungsmittelfreien Verfahren deutlich niedriger liegen.

Häufig wird die Halex-Reaktion von weiteren Nebenreaktionen begleitet, in deren Verlauf es zur Bildung signifikanter Mengen an Nebenprodukten, vor allem an reduktiv dehalogenierten Aromaten, kommt, deren Abtrennung vom Produkt wegen ähnlicher Siedepunkte äußerst schwierig und sehr teuer ist. Aufgrund dieser Nebenreaktionen ist das Einsatzgebiet für die Halex-Reaktion relativ begrenzt.

Zum näheren druckschriftlichen Stand der Technik werden
D1 = US 4,287,374
D2 = WO 87/04194
D3 = Clark et al., Tetrahedron Letters 28 [1987], 111 ff.
D4 = C.A. 109:92451t = JP 63 39,824
D5 = JP 05194303 A2 und
D6 = JP 08092148 A2
genannt.

Der Einsatz von Phasentransfer-Katalysatoren gehört bereits zum Stand der Technik, um einige der oben angesprochenen Probleme zu umgehen. Andere Probleme, wie eine schlechte Rührbarkeit der Reaktionssuspension bei lösungsmittelfreien Verfahren, bleiben jedoch weiter bestehen.

Die D1 lehrt den Einsatz von quartären Ammonium- oder Alkylphosphoniumsalzen als Phasentransfer-Katalysatoren. Gemäß der D2 werden Pyridiniumsalze, gemäß der D3 Kronenether oder Tetraphenylphosphoniumsalze als Phasentransfer-Katalysatoren verwendet. Diese Phasentransfer-Katalysatoren weisen zum Teil vergleichsweise geringe Aktivität auf und sind unter den für die Durchführung der Reaktion erforderlichen Temperaturen nur mäßig stabil.

Gemäß der D4 wird der Chlor-Fluor-Austausch in Gegenwart von Polymerisationsinhibitoren wie z.B Dinitrobenzol vorgeschlagen. Unerfreulicherweise hat die Anwendung von Dinitrobenzol jedoch einige gravierende Nachteile. Wie vor kurzem bekannt geworden ist reagiert Dinitrobenzol mit KF unter NO₂-Substitution und Bildung von Nitrit-Anionen, was zur Bildung von Phenolderivaten führt und zusätzliche Mengen von Fluorierungsreagenzien verbraucht.

Gemäß der D5 wird die Chlor-Fluor-Austauschreaktion in Nitrobenzol als Lösungsmittel durchgeführt, so daß die Raum/Zeit Ausbeute im Vergleich zu lösungsmittelfreien Verfahren deutlich niedriger liegen. Außerdem kann die Abtrennung der Produkte von Nitrobenzol schwierig sein, besonders für Verbindungen mit ähnlichen Siedepunkten.

Aus der D6 ist eine Verfahrensweise bekannt, bei welcher die Dehalogenierung von monochlorierten Benzaldehyden bei der Halex-Reaktion vermindert wird. 4-Chlorbenzaldehyd wird mit Sulfolan, KF und Tetraphenylphosphoniumbromid in Gegenwart von Nitrobenzol oder Nitronaphthalin umgesetzt. Hierdurch wird zwar die Geschwindigkeit der Bildung des Nebenprodukts Benzaldehyd verringert, die gebildete Menge ist mit 0,72% nach 3 h allerdings immer noch zu hoch. Außerdem ist die Abtrennung der Dehalogenierungsprodukte von den angestrebten fluorierten Zielprodukten im allgemeinen aufgrund sehr ähnlicher Siedepunkte schwierig.

Angesichts des hierin angegebenen und diskutierten Standes der Technik war es mithin Aufgabe der Erfindung, ein Verfahren der eingangs erwähnten Gattung anzugeben, welches die Herstellung definierter Zielverbindungen in guter Ausbeute mit hoher Selektivität und Reinheit erlaubt. Das neue Verfahren soll für den großtechnischen Einsatz tauglich sein, möglichst wenig umweltbelastend und gleichzeitig mit relativ einfachen Mitteln kostengünstig zu realisieren sein. Dabei soll das Verfahren insbesondere von den oben erwähnten Nachteilen, die bislang den im Stand der Technik vorhandenen Verfahren anhaften, möglichst weitgehend frei sein.

Weiters ist es Aufgabe der Erfindung die Halex-Reaktion so zu verbessern, daß die Dehalogenierung möglichst weit zurückgedrängt wird.

Gelöst werden diese sowie weitere nicht näher aufgezählte, jedoch aus der einleitenden Erörterung des Standes der Technik ableitbare oder erschließbare Aufgaben durch ein Verfahren der eingangs erwähnten Art mit den Merkmalen des kennzeichnenden Teils des Anspruches 1. Vorteilhafte Modifikationen des Verfahrens der Erfindung werden in den von Anspruch 1 abhängigen Unteransprüchen unter Schutz gestellt.

Dadurch, daß man bei einem Verfahren zur Herstellung von Fluor enthaltenden Verbindungen, eine Verbindung, die eine oder mehrere gegen Fluor austauschbare Halogenatome aufweist, mit einem Fluorid der allgemeinen Formel 1 oder einem Gemisch von Fluoriden der allgemeinen Formel I

KAT⁺F⁻ (I),

worin KAT⁺ für Alkalimetallion, NH₄⁺, Erdalkalimetallion oder einen Rest der allgemeinen Formel II

A¹A²A³A⁴N⁺ (II),

steht,
wobei A¹, A², A³, A⁴ unabhängig voneinander, gleich oder verschieden sind und geradkettiges oder verzweigtes Alkyl oder Alkenyl mit 1 bis 12 Kohlenstoffatomen, Cycloalkyl mit 4 bis 8 Kohlenstoffatomen, Aryl mit 6 bis 12 Kohlenstoffatomen oder Aralkyl mit 7 bis 12 Kohlenstoffatomen bedeuten, in Anwesenheit einer Verbindung oder eines Gemisches von Verbindungen der allgemeinen Formel III 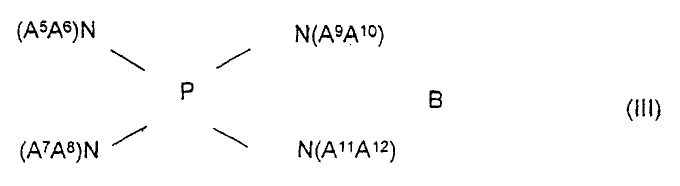 worin, A⁵, A⁶, A⁷, A⁸, A⁹, A¹⁰, A¹¹, A¹² unabhängig voneinander, gleich oder verschieden sind und geradkettiges oder verzweigtes Alkyl oder Alkenyl mit 1 bis 12 Kohlenstoffatomen, Cycloalkyl mit 4 bis 8 Kohfenstoffatomen, Aryl mit 6 bis 12 Kohlenstoffatomen, Aralkyl mit 7 bis 12 Kohlenstoffatomen bedeuten, oder A⁵A⁶, A⁷A⁸, A⁹A¹⁰, A¹¹A¹² unabhängig voneinander, gleich oder verschieden sind und direkt oder über O oder N-A¹³ miteinander zu einem Ring mit 3 bis 7 Ringgliedern verbunden sind, A¹³ für ein Alkyl mit 1 bis 4 Kohlenstoffatomen steht und B⁻ einen einwertigen Säurerest oder das Äquivalent eines mehrwertigen Säurerestes bedeutet, und in Gegenwart einer oder mehrerer Verbindungen der allgemeinen Formeln IV, umfaßend IVa und/oder IVb

X-NO₂ (IVa),

X-SO-X' (IVb),

worin X und X' unabhängig voneinander gleich oder verschieden für (C₆-C₁₈)-Aryl, unsubstituiert oder substituiert, für (C₆-C₁₈)-Aryioxy, substituiert oder unsubstituiert, (C₆-C₁₈)-Arylthio, substituiert oder unsubstituiert, (C₇-C₁₂)-Aralkyl, substituiert oder unsubstituiert, oder einen Rest der Formel V stehen 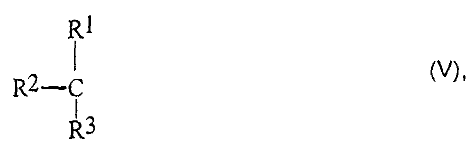 worin R¹, R², R³ unabhängig voneinander, gleich oder verschieden sind und für Wasserstoff, geradkettiges oder verzweigtes Alkyl oder Alkenyl mit 1 bis 12 Kohlenstoffatomen, Cycloalkyl mit 4 bis 8 Kohlenstoffatomen, für Aryl, substituiertes Aryl, Aryloxy, Arylthio, jeweils mit 6 bis 18 Kohlenstoffatomen, oder Aralkyl mit 7 bis 12 Kohlenstoffatomen stehen, in Anwesenheit oder Abwesenheit eines Lösungsmittels bei Temperaturen im Bereich von 40 °C bis 260 °C umsetzt, gelingt es besonders vor-teilhaft ein Verfahren bereitzustellen, welches die bekannten Verfahren hinsichtlich der Selektivität und der Qualität der resultierenden Verfahrensprodukte in nicht ohne weiteres vorhersehbarer Weise verbessert.

Alkalimetallion bedeutet Lithium, Natrium, Kalium, Rubidium und/oder Cäsium, insbesondere Natrium und/oder Kalium, ganz besonders Kalium;

Erdalkalimetallion bedeutet Magnesium, Calcium, Strontium und/oder Barium, insbesondere Calcium;

Alkyl mit 1 bis 4 Kohlenstoffatomen umfaßt geradkettige oder verzweigte Reste, wie z.B. die Methyl-, Ethyl-, n-Propyl-, Isopropyl-, n-Butylgruppe und tert.-Butyl.

Geradkettiges Alkyl oder Alkenyl mit 1 bis 12 Kohlenstoffatomen umfaßt unter anderem unverzweigte, gesättigte Kohlenwasserstoffreste, wie z.B. die Methyl-, Ethyl-, Propyl-, Butyl-, Pentyl-, Hexyl-, Heptyl-, Octyl-, Nonyl-, Decyl-, Undecyl- und Dodecylgruppe, sowie unverzweigte, ungesättigte Kohlenwasserstoffreste wie die Vinyl-, Allyl-, 2-Butenyl-, 2-Pentenyl- und 2-Decenylgruppe.

Geradkettiges oder verzweigtes Alkyl oder Alkenyl mit 1 bis 12 Kohlenstoffatomen umfaßt unter anderem die vorgenannten geradkettigen Alkyle oder Alkenyle sowie verzweigte Reste wie die Isopropyl-, 2-Butyl-, 2-Methylpropyl-, tert.-Butyl-, 2-Methylbutyl-, 1,1-Dimethylpropyl-, 1,1,3,3-Tetramethylbutyl- und 2-Decylgruppe.

Cycloalkyl mit 4 bis 8 Kohlenstoffatomen bedeutet Cyclobutylrest, Cyclopentylrest, Cyclohexylrest, Cycloheptylrest oder Cyclooctylrest, vorzugsweise Cyclohexylrest. Cycloheptylrest oder Cyclooctylrest, ganz besonders bevorzugt Cyclohexylrest.

Unter dem Ausdruck "Aryl" wird ein cyclischer aromatischer Rest mit 6 bis 18, insbesondere 6 bis 14 Kohlenstoffatomen, ganz besonders bevorzugt mit 6 bis 12 Kohlenstorfatomen, wie beispielsweise Phenyl, Naphthyl oder Biphenyl, vorzugsweise Phenyl, verstanden.

(C₆-C₁₈)-Aryl, unsubstituiert oder substituiert umfaßt zunächst unsubstituierte Aryle wie vorgenannt; diese können einfach oder mehrfach, bis zu 3-fach substituiert sein; als Substituenten kommen grundsätzlich in Frage: F, NO₂ (nicht bei Verbindungen der allgemeinen Formeln I, II, III), CF₃, CN, CHO, COF, SO₂F, OCF₃, SOCF₃, SO₂CF₃, COOR, CONRR', SO₂R, COR oder OR oder einen Rest -OC-NR-CO- beziehungsweise -OC-O-CO-, der zwei ortho-Steliungen verknüpft, aufweist, wobei R und R' jeweils unabhängig voneinander, gleich oder verschieden sind, und für Wasserstoff, ein geradkettiges oder verzweigtes Alkyl mit 1 bis 4 Kohlenstoffatomen, ein Aryl mit 6 bis 12 Kohlenstoffatomen oder Aralkyl mit 7 bis 12 Kohlenstoffatomen stehen einfach bis dreifach fluorsubstituiert sind, und R und R' gegebenenfalls zu einem drei- bis siebengliedrigen Ring verbunden sind;

(C₆-C₁₈)-Aryloxy, substituiert oder unsubstituiert, umfaßt zunächst Aryloxy mit 6 bis 18 Kohlenstoffatomen, vorzugsweise isocyclische Verbindungen; die unsubstituierten Aryloxy-Reste sind beispielsweise die Phenoxy- oder 1- oder 2-Naphthyloxygruppe; diese können wie die substituierten Aryle entsprechende Reste als Substituenten aufweisen;

(C₆-C₁₈)-Arylthio, substituiert oder unsubstituiert, umfaßt zunächst Thioaryle mit 6 bis 18 Kohlenstoffatomen, besonders bevorzugt isocyclische Verbindungen; die unsubstituierten Arylthio-Reste sind beispielsweise die Phenylthio- oder 1- oder 2-Naphthylthiogruppe; diese können wie die substituierten Aryle entsprechende Reste als Substituenten aufweisen;

(C₇-C₁₂)-Aralkyl, substituiert oder unsubstituiert, umfaßt Aralkyle mit 7 bis 12 Kohlenstoffatomen; hierzu gehören neben anderen die Benzyl-, 2-Phenylethyl-, 1-Phenylethyl-, 1-Methyl-1-phenylethyl-, 3-Phenylpropyl-, 4-Phenylbutyl, 2-Methyl-2-phenylethylgruppe oder die 1-Methyl- oder 2-Methylnaphthylgruppe. Hinsichtlich der möglichen Substitution gilt, daß diese Gruppen Kern- und/oder Seitengruppensubstituenten aufweisen können; als Substituenten am Kern kommen unter anderem bis zu drei der folgenden Reste in Frage: F, NO₂ (nicht bei Verbindungen der allgemeinen Formeln I, II, III), CF₃, CN, CHO, COF, SO₂F, OCF₃, SOCF₃, SO₂CF₃, COOR, CONRR', SO₂R, COR oder OR oder einen Rest -OC-NR-CO- beziehungsweise -OC-O-CO-, der zwei ortho-Stellungen verknüpft, aufweist, wobei R und R' jeweils unabhängig voneinander, gleich oder verschieden sind, und für Wasserstoff, ein geradkettiges oder verzweigtes Alkyl mit 1 bis 4 Kohlenstoffatomen, ein Aryl mit 6 bis 12 Kohlenstoffatomen oder Aralkyl mit 7 bis 12 Kohlenstoffatomen stehen einfach bis dreifach fluorsubstituiert sind, und R und R' gegebenenfalls zu einem dreibis siebengliedrigen Ring verbunden sind.

Insbesondere und unter anderem vereinigt das erfindungsgemäße Verfahren eine Reihe von außerordentlichen Vorteilen in sich:
- Es ist als überraschend anzusehen, daß es durch die Verwendung der Verbindung der allgemeinen Formeln IV (als Zusatz zum Reaktionsgemisch für die Chlor-Fluor-Austauschreaktion) zusammen mit den Phasentransferkatalysatoren der allgemeinen Formel III, gelingt, die Bildung unerwünschter Nebenprodukte (vor allem reduktiv dehalogenierter Aromaten) zurückzudrängen oder vollständig zu vermeiden.
- Unter anderem ist dadurch der Einsatz von Verbindungen der allgemeinen Formeln IV in Kombination mit Verbindungen der allgemeinen Formel III als Zusatz für die Chlor-Fluor-Austauschreaktion insgesamt gesehen ein umweltfreundlicher chemischer Prozeß.

Die Verbindungen der allgemeinen Formeln IV und III können als Feststoffe oder Flüssigkeiten ein breites Spektrum von Schmelzpunkten oder Siedepunkten abdecken, so daß durch gezielte Auswahl einer geeigneten Verbindung in Abhängigkeit vom Siedepunkt des erwarteten Zielprodukts in fast allen Fällen eine Aufarbeitung des Reaktionsgemisches nach der Fluorierung durch destillative Auftrennung des Reaktionsgemisches erfolgen kann.
- Hierdurch ist es ferner möglich, auch die Verbindungen der Formeln IV und/oder III zu isolieren und wieder zu recyclieren.
- Die Nitro- und Thioxo-Verbindungen der allgemeinen Formeln IV können im Rahmen der Erfindung diese Vorteile bereits dann zur Verfügung steilen, wenn sie im Reaktionsgemisch enthalten sind. D.h., daß sie einen Zusatz zum Reaktionsgemisch für die Fluorierung mittels Halogenaustausch darstellen. Der Anteil an Verbindung der allgemeinen Formeln IV ist variabel, da schon kleine Zusätze eine insgesamt positive Beeinflussung der Halex-Reaktion zu gewährleisten in der Lage sind.
- Die als Zusatz erfindungsgemäß in Frage kommenden Verbindungen der allgemeinen Formeln IV und III sind häufigenfalls sehr billig und zum überwiegenden Teil kommerziell erhältlich und damit verfügbar. Verbindungen der allgemeinen Formel IV oder III, welche nicht käuflich verfügbar sind, lassen sich auf einfache Weise nach dem Fachmann geläufigen Verfahren synthetisieren.

Die mit der Erfindung erzleibaren vorteilhaften Effekte stellen sich ein, wenn der Halex-Reaktion eine Verbindung der allgemeinen Formel IVa und/oder IVb oder eine Mischung von mehreren Verbindungen der allgemeinen Formeln IV (umfassend die Formeln IVa und/oder IVb), jeweils zusammen mit wenigstens einem Katalysator der Formel III, zugesetzt werden.

Die Menge der Verbindungen der allgemeinen Formeln IV, die erfindungsgemäß eingesetzt werden, kann über einen weiten Bereich variieren. Im allgemeinen kann man recht brauchbare Ergebnisse erzielen, wenn man in Bezug auf die einzusetzende auszutauschendes Halogen aufweisende Verbindung etwa 0,1 bis 20 Gew.-% einsetzt. Liegt die Menge unterhalb von 0,1 Gew.-% ist die Reduzierung des Auftretens der Dehalogenierungsprodukte nicht ausgeprägt genug. Liegt die Menge an Verbindung der Formeln IV über 20 Gew.-%, wird im allgemeinen kein meßbar besserer Effekt erzielt als mit geringeren Zusätzen. Bevorzugt sind Zusätze im Bereich von 0,5 bis 10 Gew.-%. Besonders zweckmäßig führt man das Verfahren der Erfindung in Gegenwart von 1 bis 5 Gew.-% an einer oder mehreren Verbindungen der allgemeinen Formeln IVa und/oder IVb durch, bezogen auf das Gewicht der Edukte (austauschbares Halogen aufweisende Verbindungen).

Die Verbindungen der allgemeinen Formeln IV. welche zwei oder mehr Nitrogruppen, zwei oder mehr Thioxogruppen, eine Nitrogruppe und eine Thioxogruppe, eine Nitrogruppe und mehrere Thioxogruppen oder eine Thioxogruppe und mehrere Nitrogruppen enthalten, können in kleineren Mengen zugesetzt werden, verglichen mit Mononitroderivaten oder Monothioxoverbindungen. Zweckmäßig ist hierbei ein Anteil von 0,1 - 10 Gew.-%, bevorzugt 0,5 - 8 Gew.-%, insbesondere 1 - 5 Gew.-% bezogen auf die eingesetzten Edukte.

Die Nitroverbindungen der allgemeinen Formel IVa und die Thioxoverbindungen der allgemeinen Formel IVb umfassen grundsätzlich aromatische Verbindungen und aliphatische Verbindungen. Die aromatischen Verbindungen wiederum können mit der Nitrogruppe und/oder Thioxogruppe kernsubstituiert sein, die Nitrogruppe und/oder Thioxogruppe kann sich bei der aromatischen Verbindung auch in einer Seitengruppe befinden.

Zu den aromatischen Verbindungen gehören im Rahmen der Erfindung diejenigen Nitroverbindungen der allgemeinen Formel IVa und/oder Thioxoverbindungen der allgemeinen Formel IVb, worin X für (C₆-C₁₈)-Aryl, unsubstituiert oder substituiert, für (C₆-C₁₈)-Aryloxy, substituiert oder unsubstituiert, (C₆-C₁₈)-Arylthio, substituiert oder unsubstituiert, oder (C₇-C₁₂)-Aralkyl, substituiert oder unsubstituiert, steht.

Bevorzugt hiervon sind unsubstituierte oder substituierte Aryle mit 6 bis 8 Kohlenstoffatomen, unsubstituierte oder substituierte Aryloxy-Reste mit 6 bis 8 Kohlenstoffatomen, unsubstituierte oder substituierte Arylthio-Reste mit 6 bis 8 Kohlenstoffatomen und unsubstituierte oder substituierte Aralkyle mit 8 bis 10 Kohlenstoffatomen.

Von ganz besonderem Interesse sind unsubstituierte oder substituierte Nitro-Aryle mit 6 bis 8 Kohlenstoffatomen und unsubstituierte oder substituierte Nitro-Aryloxy-Verbindungen mit 6 bis 8 Kohlenstoffatomen.

Unter den aliphatischen Nitroverbindungen und/oder Thioxoverbindungen sind im Zusammenhang mit der Erfindung insbesondere solche zu nennen, bei denen in der Formel IV der Rest X für einen Rest der Formel V steht 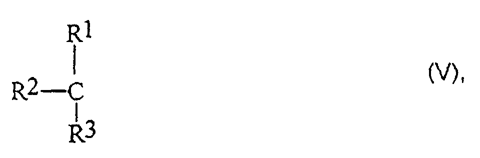 worin R¹, R², R³ unabhängig voneinander, gleich oder verschieden sind und für Wasserstoff, geradkettiges oder verzweigtes Alkyl oder Alkenyl mit 1 bis 12 Kohlenstoffatomen oder Cycloalkyl mit 4 bis 8 Kohlenstoffatomen stehen.

Besonders vorteilhaft ist der Einsatz von Verbindungen der Formeln IV, worin der Rest X für einen Rest der Formel V steht, worin R¹, R², R³ unabhängig voneinander, gleich oder verschieden sind und für Wasserstoff, geradkettiges oder verzweigtes Alkyl mit 1 bis 4 Kohlenstoffatomen oder Cycloalkyl mit 5 bis 7 Kohlenstoffatomen, vorzugsweise Cxclohexyl, stehen.

Zu bevorzugt erfindungsgemäß einzusetzenden Verbindungen gehören unter anderem Nitrobenzol, 2-Fluoronitrobenzol, 3-Fluoronitrobenzol, 4-Fluoronitrobenzol, 2,4-Difluoronitrobenzol, 3-Chlornitrobenzol, 2-Nitrotoluol, 3-Nitrotoluol, 4-Nitrotoluol, 2-Nitroanisol, 3-Nitroanisol, 4-Nitroanisol, 2-Nitrothiophen, 4-Nitro-2-propylbenzol, 1-Nitronaphtalin, 2-Nitronaphtalin, 2,4-Dinitrodiphenyl, 4,4'-Dinitrodiphenyl, 4,4'Dinitrodiphenylether, Dinitrodiphenyldisulfid, Nitromethan, Nitroethan, Nitropropan, Nitroanthracen, 1-Nitropyren, Dimethylsulfoxid, Diphenylsulfoxid, Phenylmethylsulfoxid, Diethylsulfoxid und/oder Methyltrifluoromethylsulfoxid.

Ganz besonders bevorzugt aufgrund ihres günstigen Preises sowie der universellen Verfügbarkeit sind Nitrobenzol und/oder Dimethylsulfoxid (DMSO).

Ein großer Vorteil des Verfahrens der Erfindung besteht in seiner universellen Anwendbarkeit auf eine Vielzahl von Substraten, die ein oder mehrere gegen Fluor austauschbare Halogenatome aufweisen.

Dabei wird unter dem Begriff "gegen Fluor austauschbares Halogen" Chlor, Brom oder Jod, insbesondere Chlor oder Brom, bevorzug Chlor, das durch Fluorid im Sinne einer nucleophilen Substitution gegen Fluor ausgetauscht werden kann, verstanden.

Das Spektrum der erfindungsgemäß in Gegenwart der Verbindungen der Formeln III sowie IVa und/oder IVb umzusetzenden Substrate ist äußerst breit und umfangreich.

So ist es bevorzugt, als Verbindung, die gegen Fluor austauschbares Halogen enthält, eine am Kern einen gegen Fluor austauschbaren Chlor- oder Bromsubstituenten, insbesondere Chlorsubstituenten, besitzende aromatische Verbindung mit 0 bis 3 Stickstoffatome im Kern, die gegebenenfalls wenigstens einen weiteren, die nucleophile Substitution der aromatischen Verbindungen begünstigenden Substituenten aufweist, einzusetzen.

Dabei ist das erfindungsgemäße Verfahren gleichermaßen gut auf aromatische oder heteroaromatische Verbindungen anwendbar. Ebenso ist die Fluorierung bei zyklischen Verbindungen mit nur einem Ring wie auch bei kondensierten zyklischen Verbindungen und heterozyklischen Verbindungen einsetzbar. Ohne Anspruch auf Vollständigkeit zu erheben kommen als Ausgangsverbindungen solche bevorzugt in Frage, die eine oder mehrere gegen Fluor austauschbare Halogenatomen aufweisen und vom Benzol-, Naphthalin-, Pyridin-, Anthracen-, Phenanthren-, Pyrimidinund Pyrazin-Typ sowie vom Typ benzoanellierter Ringsysteme des Pyridins (Chinotin-, Isochinolin-, Acridin-, Acridon-Typ), des Pyrimidins, Pyrazins und Piperazins (Benzodiazine des Cinnolin-, Phthalazin-, Chinazolin-, Chinoxalin-, Phenazin-, Phenoxazin-Typs) sind. Ebenso kommen deren Derivate in Betracht, die gegebenenfalls wenigstens einen weiteren, die nucleophile Substitution an aromatischen Verbindungen begünstigenden Substituenten aufweisen. Dieser weitere, die nucleophile Substitution der aromatischen Verbindung begünstigende Substituent führt üblicherweise zu einer Aktivierung der aromatischen Verbindung, die dadurch einer Halogen-Fluor-Austauschreaktion leichter zugänglich wird.

Bei dem weiteren, die nucleophile Substitution an einer aromatischen Verbindung begünstigenden Substituenten handelt es sich um -I- und -M-Substituenten, welche die Elektronendichte respektive Nucleophilie des Aromaten herabsetzen und dadurch eine elektrophile Substitution erschweren. Der Aromat wird dadurch jedoch gegenüber einer nucleophilen Substitution aktiviert. Die aktivierende Wirkung dieser Substituenten ist besonders groß, wenn sie in ortho- oder para-Stellung zu dem gegen Fluor auszutauschenden Halogen stehen.

In einer wertvollen Ausführungsform setzt man eine am Kern ein gegen Fluor austauschbares Halogenatom besitzende aromatische Verbindung um, die wenigstens einen weiteren Substituenten aus der Reihe F, Cl, Br, J, CF₃, CN, CHO, COF, COCl, SO₂F, SO₂Cl, OCF₃, SOCF₃, SO₂CF₃, COOR, CONRR', SO₂R, COR oder OR oder einen Rest -OC-NR-CO- beziehungsweise -OC-O-CO-, der zwei ortho-Stellungen verknüpft, aufweist, wobei R und R' jeweils unabhängig voneinander, gleich oder verschieden sind, und für Wasserstoff, ein geradkettiges oder verzweigtes Alkyl mit 1 bis 4 Kohlenstoffatomen, ein Aryl mit 6 bis 12 Kohlenstoffatomen oder Aralkyl mit 7 bis 12 Kohlenstoffatomen stehen und die Alkyle und Aralkyle gegebenenfalls einfach bis dreifach halogensubstituiert sind, und R und R' gegebenenfalls zu einem dreibis siebengliedrigen Ring verbunden sind.

Man kann auch eine am Kern einen gegen Fluor austauschbaren Halogensubstituenten besitzende aromatische Verbindung einsetzen, die wenigstens einen weiteren Substituenten aus der Reihe F, Cl, Br, J, CF₃, CN, CHO, COF, COCl, SO₂F, SO₂Cl, OCF₃, SOCF₃, SO₂CF₃, COOR, CONRR', SO₂R, COR oder OR oder einen Rest -OC-NR-CO- beziehungsweise -OC-O-CO-, der zwei ortho-Stellungen verknüpft, aufweist, wobei R und R' jeweils unabhängig voneinander, gleich oder verschieden sind, und für Wasserstoff, ein geradkettiges oder verzweigtes Alkyl mit 1 bis 6 Kohlenstoffatomen. ein Aryl mit 6 bis 12 Kohlenstoffatomen oder Aralkyl mit 7 bis 12 Kohlenstoffatomen stehen und die Alkyle und Aralkyle gegebenenfalls einfach bis dreifach halogensubstituiert sind.

Die vorstehend erwähnten aromatischen Verbindungen können auch zusätzliche Substituenten enthalten, beispielsweise Alkylreste, Aminogruppen, Alkylaminogruppen, Hydroxygruppen oder Alkoxygruppen.

Man kann auch als Ausgangssubstrat eine am Kern einen gegen Fluor austauschbaren Halogensubstituenten besitzende aromatische Verbindung einsetzen, die wenigstens einen weiteren gegen Fluor austauschbaren Halogensubstituenten und gegebenenfalls einen weiteren Substituenten aus der Reihe F, CF₃, CN, CHO, COF, COCl, SO₂F, SO₂Cl, OCF₃, SOCF₃, SO₂CF₃, COOR, CONRR', SO₂R, COR oder OR oder einen Rest -OC-NR-CO- beziehungsweise -OC-O-CO-, der zwei ortho-Stellungen verknüpft, aufweist. Diese Ausgangsverbindungen besitzen demzufolge wenigstens zwei gegen Fluor austauschbare Halogenatome. Diese Substrate sind üblicherweise einem ein- oder zweifachen Halogen-Fluor-Austausch zugänglich, ohne daß sie einen weiteren Substituenten aus der vorstehend genannten Reihe besitzen müssen. Sie können aber auch einen weiteren, die nucleophile Substitution der aromatischen Verbindung begünstigenden Substituenten aus der Reihe der zuvor genannten Reste besitzen. Die Anwesenheit der Substituenten erhöht die Reaktivität der aromatischen Verbindung hinsichtlich der Halogen-Fluor-Austauschreaktion.

Der Einbau wenigstens eines Stickstoffatoms in den aromatischen Ring erhöht die Reaktivität der aromatischen Verbindung derart, daß gegebenenfalls auch ohne Anwesenheit eines weiteren, die nucleophile Substitution der aromatischen Verbindung begünstigenden Substituenten ein Halogen-Fluor-Austausch stattfinden kann.

Mit gutem Erfolg werden erfindungsgemäß auch Verbindungen der allgemeinen Formel VI 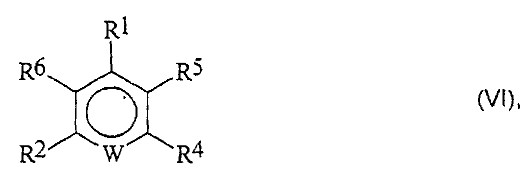 worin W für N oder C-R³ steht, einer der Reste R¹, R², R⁴, R⁵, R⁶ und gegebenenfalls R³ F, Cl, CF₃, CN, CHO, COF, COCl, SO₂F, SO₂Cl, OCF₃,SO₂CF₃, COOR, CONRR', SO₂R, COR oder OR ist oder zwei der Reste, die in ortho-Stellung zueinander stehen, -CO-O-CO- oder -CO-NR-CO- bedeuten, wobei R und R' jeweils unabhängig voneinander, gleich oder verschieden sind und für Wasserstoff, ein geradkettiges oder verzweigtes Alkyl mit 1 bis 6 Kohlenstoffatomen, ein Aryl mit 6 bis 12 Kohlenstoffatomen oder Aralkyl mit 7 bis 12 Kohlenstoffatomen stehen, ein weiterer der Reste R¹, R², R⁴, R⁵, R⁶ und gegebenenfalls R³ Halogen ist und die übrigen Reste für Wasserstoff, F oder Cl stehen, eingesetzt.

Die Reste -CO-O-CO- und -CO-NR-CO- betreffen allgemein zwei der Reste R¹ bis R⁶, die in ortho-Stellung zueinander stehen, insbesondere zwei in ortho-Stellung zueinander stehende Reste aus der Gruppe R¹, R², R⁴, R⁵, R⁶, falls W für N steht, oder zwei in ortho-Stellung zueinander stehende Reste aus der Gruppe R², R³ und R⁴, falls W für C-R³ steht.

In der Verbindung der Formel VI steht einer der Reste R¹, R², R⁴, R⁵, R⁶ und gegebenenfalls R³ oder der Rest R³ insbesondere für F, Cl, CF₃, CN, CHO, COF, COCl, OCF₃, COOR, CONRR', COR, OR, -CO-O-CO- oder -CO-NR-CO-, bevorzugt für Cl, F, CF₃, CN, CHO, COOR oder COCl; R und R' bedeuten insbesondere Wasserstoff, ein geradkettiges oder verzweigtes Alkyl mit 1 bis 4 Kohlenstoffatomen oder Aryl mit 6 bis 12 Kohlenstoffatomen, bevorzugt Wasserstoff oder ein geradkettiges oder verzweigtes Alkyl mit 1 bis 3 Kohlenstoffatomen, besonders bevorzugt Methyl oder Ethyl; einer oder zwei der Reste R¹, R², R⁴, R⁵, R⁶ und gegebenenfalls R³ stehen für Halogen und die übrigen Reste sind gleich oder verschieden und stehen für H oder F.

Eine besonders bevorzugte Substratgruppe, mit welcher das Verfahren gemäß der Erfindung zu sehr guten Ergebnissen führt, ist die Gruppe der substituierten Benzaldehyde und Benzonitrile. Hiervon wiederum sind 2-Chlorbenzaldehyd, 3-Chlor-benzaldehyd, 4-Chlorbenzaldehyd, 2-Brombenzaldehyd, 3-Brombenzaldehyd, 4-Brombenzaldehyd, 2,3-Dichlorbenzaldehyd, 2,4-Dichlorbenzaldehyd, 2,6-Dichlorbenzaldehyd, 3,5-Dichlorbenzaldehyd, 2,4,6-Trichlorbenzaldehyd, 2-Chlor-benzonitril, 3-Chlorbenzonitril, 4-Chlorbenzonitril, 2-Brombenzonitril, 3-Bromben-zonitril, 4-Brombenzonitril, 2,3-Dichlorbenzonitril, 2,4-Dichlorbenzonitril, 2,6-Dichlor-benzonitril, 3,5-Dichlorbenzonitril und 2,4,6-Trichlorbenzonitril ganz besonders bevorzugte Substrate.

Zur Durchführung der erfindungsgemäßen Reaktion setzt man ein Fluorid der allgemeinen Formel I oder ein Gemisch von Fiuoriden der allgemeinen Formel I

KAT⁺F⁻ (I)

ein, worin KAT⁺ für Alkalimetallion, NH₄⁺, Erdalkalimetallion oder einen Rest der allgemeinen Formel II

A¹A²A³A⁴N⁺ (II),

steht, wobei A¹, A², A³, A⁴ unabhängig voneinander, gleich oder verschieden sind und ein geradkettiges oder verzweigtes Alkyl oder Alkenyl mit 1 bis 12 Kohlenstoffatomen, Cycloalkyl mit 4 bis 8 Kohlenstoffatomen, Aryl mit 6 bis 12 Kohlenstoffatomen oder Aralkyl mit 7 bis 12 Kohlenstoffatomen bedeuten.

Bevorzugt ist in diesem Zusammenhang der Einsatz von Kalziumfluorid, Ammoniumfluorid, Lithiumfluorid, Natriumfluorid, Kaliumfluorid, Rubidiumfluorid, Cäsiumfiuorid, oder ein Gemisch derselben, insbesondere Lithiumfluorid, Natriumfluorid, Kaliumfluorid, Rubidiumfluorid, Cäsiumfluorid oder ein Gemisch derselben, zweckmäßig Natriumfluorid, Kaliumfluorid, Cäsiumfluorid oder ein Gemisch derselben, besonders bevorzugt Kaliumfluorid und/oder Cäsiumfluorid, Häufig genügt es, Kaliumfluorid als alleiniges Fluorid zu verwenden.

Unter den Resten der allgemeinen Formel II sind wiederum solche besonders vorteilig, welche die Fluorierung durch Halex-Reaktion mit einer oder mehreren Verbindungen aus der Gruppe umfassend Tetramethylammoniumfluorid, Tetraethylammoniumfluorid, Tetrapropylammoniumfluorid, Tetra(n-butyl)ammoniumhydrogenfluorid und/oder Tetraphenylammoniumfluorid ermöglichen. Besonders günstig sind Tetramethylammoniumfluorid und/oder Tetraphenylammoniumfluorid.

Die Fluorierungsmittel der Formel I werden im Rahmen der Erfindung in einer Menge eingesetzt, die ausreicht, das gewünschte Maß des Halogenaustauschs zu erreichen. Bevorzugt ist ihr stöchiometrischer Einsatz bezogen auf die Menge der Ausgangsverbindung. Bevorzugt ist auch ein Einsatz im Überschuß, besonders bevorzugt die 1,1 bis 2,0-fache molare Menge bezogen auf die Mol der zu ersetzenden Halogenatome in der oder den Ausgangsverbindungen.

Was das Mengenverhältnis betrifft, so ist jedoch zu berücksichtigen, daß es Fälle geben kann, in denen ein Überschuß an Fiuorid zu unerwünschten Nebenprodukten führen kann. In diesen Fällen empfiehlt es sich, die Fluoride der Formel I auch im Unterschuß einzusetzen.

Man setzt üblicherweise das Fiuorid der Formel I : Äquivalent auszutauschende Halogenatome im Verhältnis (0,5 bis 10): 1, insbesondere (0,8 bis 5): 1, besonders bevorzugt (1 bis 1,5): 1, ein.

Wie eingangs bereits erwähnt, führt man die Umsetzung in Anwesenheit einer Verbindung der Formel III, die als Katalysator fungiert, durch.

Die Verbindungen der allgemeinen Formel III lassen sich beispielsweise durch Umsetzung von Phosphorpentachlorid mit Dialkylaminen herstellen. Man kann aber auch Phosphorpentachlorid stufenweise mit unterschiedlichen sekundären Aminen, beispielsweise Dialkylaminen, zur Reaktion bringen, um unsymmetrisch substituierte Verbindungen der Formel III zu erhalten. Weitere Möglichkeiten, Verbindungen der Formel III zu synthetisieren, sind von R. Schwesinger et al., Angew. Chemie 103 (1991) 1376 und R. Schwesinger et al., Chem. Ber. 127 (1994) 2435 bis 2454 beschrieben. Die Verbindungen sind daher nach dem Fachmann bekannten Verfahren gut erhältlich.

Günstig ist es eine Verbindung der allgemeinen Formeln III einzusetzen, worin A⁵, A⁶, A⁷, A⁸, A⁹, A¹⁰, A¹¹, A¹², unabhängig voneinander, gleich oder verschieden sind und für geradkettiges oder verzweigtes Alkyl oder Alkenyl, insbesondere Alkyl, mit 1 bis 12 Kohlenstoffatomen, insbesondere 1 bis 8 Kohlenstoffatomen, bevorzugt 1 bis 4 Kohlenstoffatomen, oder Cycloalkyl mit 4 bis 8 Kohlenstoffatomen, insbesondere 5. bis 6 Kohlenstoffatomen, stehen. Diese Verbindungen sind von besonderem Interesse, da sie sich auf vergleichsweise einfache Art ausgehend von den entsprechenden Dialkylaminen; Dialkylenaminen, Dicycloalkylaminen, sekundären Aminen. die einen Alkyl- und Alkenylrest, einen Alkyl- und Cycloalkylrest oder einen Alkenylund Cycloalkytrest enthalten, herstellen lassen.

Als Beispiel für Alkyl sind Methyl, Ethyl, n-Propyl, i-Propyl, n-Butyl, i-Butyl, n-Pentyl, 3-Methylbutyl, n-Hexyl, 2-Ethylhexyl, insbesondere Methyl, Ethyl, n-Propyl, n-Butyl und als Beispiele für Alkenyl sind Allyl, Prop-(2)-enyl, n-But-(2)-enyl, und als Beispiele für Cycloalkyl sind Cyclopentyl, Cyclohexyl, 4-Methylcyclohexyl, 4-tert.-Butylcyclohexyl, zu nennen.

Zweckmäßig kann man auch eine Verbindung der allgemeinen Formel III einsetzen, worin A⁵A⁶ = A⁷A⁸ oder A⁵A⁶ = A⁷A⁸ = A⁹A¹⁰ oder A⁵A⁶ = A⁷A⁸ = A⁹A¹⁰ = A¹¹A¹² ist. Diese Verbindungen, in denen zwei oder mehrere der Gruppen A⁵A⁶, A⁷A⁸, A⁹A¹⁰ und A¹¹A¹² einander gleich sind, sind relativ gut zugänglich.

Man kann auch eine Verbindung der Formel III einsetzen, worin A⁵ = A⁶, A⁷ = A⁸, A⁹ = A¹⁰ und/oder A¹¹ = A¹² ist. Diese Verbindungen sind vergleichsweise leicht zugänglich und deshalb von einigem Interesse.

In weiters bevorzugter Ausführungsform zeichnet sich das Verfahren der Erfindung dadurch aus, daß man eine Verbindung der allgemeinen Formel III einsetzt, worin A⁵ = A⁶ = A⁷ = A⁸ oder A⁵ = A⁶ = A⁷ = A⁸ = A⁹ = A¹⁰, oder A⁵ = A⁶ = A⁷ = A⁸ = A⁹ = A¹⁰ = A¹¹ = A¹² ist. Die vorstehend genannten Verbindungen, worin vier, sechs oder acht der Reste A⁵ bis A¹² gleich sind, sind aufgrund ihrer Verfügbarkeit ebenfalls von Bedeutung.

Noch eine Abwandlung des Verfahrens der Erfindung sieht vor, daß man eine Verbindung der allgemeinen Formel III einsetzt, worin A⁵A⁶ oder A⁵A⁶ und A⁷A⁸ oder A⁵A⁶ und A⁷A⁸ und A⁹A¹⁰ oder A⁵A⁶ und A⁷A⁸ und A⁹A¹⁰ und A¹¹A¹² direkt oder über O oder N-A¹³ miteinander zu einem gesättigten oder ungesättigten Ring mit 5 oder 6 Ringgliedern verbunden sind. Dementsprechend enthalten diese Verbindungen einen, zwei, drei oder vier der hierin weiter oben angesprochenen Ringe.

Es besteht außerdem die vorteilhafte Möglichkeit beim beanspruchten Verfahren, daß eine Verbindung der allgemeinen Formel eingesetzt wird, worin A⁵A⁶ oder A⁷A⁸ und A⁹A¹⁰ oder A⁵A⁶ und A⁷A⁸ und A⁹A¹⁰ oder A⁵A⁶ und A⁷A⁸ und A⁹A¹⁰ und A¹¹A¹² zu einem Ring, der das N-Atom, an dem die jeweiligen Reste A⁵ bis A¹² sitzen, und gegebenenfalls O oder N-A¹³ und CH₂-Gruppen als Ringglieder umfaßt, verbunden sind. In dieser Stoffgruppe bilden die N-Atome mit den an ihnen jeweils befindlichen Resten A¹ bis A⁸ beispielsweise einen Hexahydropyridinring, Tetrahydropyrrolring, einen Hexahydropyrazinring oder auch einen Morpholinring. Dementsprechend enthalten diese Verbindungen einen, zwei, drei oder vier der vorstehend bezeichneten Ringe.

In der Verbindung der Formel III steht B⁻, wie bereits eingangs erwähnt, für einen einwertigen Säurerest oder das Äquivalent eines mehrwertigen Säurerests, insbesondere den Rest einer anorganischen Mineralsäure, einer organischen Carbonsäure, einer aliphatischen oder aromatischen Sulfonsäure.

Üblicherweise setzt man eine Verbindung der Formel III ein, worin B⁻ für F⁻, Cl⁻, Br⁻, J⁻, HF₂⁻, BF₄⁻, C₆H₅SO₃⁻, p-CH₃-C₆H₅SO₃⁻, HSO₄⁻, PF₆⁻, CF₃SO₃⁻, insbesondere für F⁻, Cl⁻, Br⁻, J⁻, HF₂⁻, BF₄⁻ steht.

Man setzt die Verbindung der Formel III zweckmäßig in einer Menge von 0,5 bis 35, insbesondere 1 bis 30, bevorzugt 3 bis 25 Gewichtsprozent, bezogen auf die Verbindung, die gegen Fluor austauschbares Halogen enthält, ein.

Um nicht ausschließlich auf die vorstehenden Angaben in Gewichtsprozent angewiesen zu sein, kann man in einer Vielzahl von Fällen, die Verbindung der Formel III in einer Menge von 0,1 bis 5, insbesondere 0,4 bis 2, bevorzugt 0,5 bis 1 Mol-%, bezogen auf die Verbindung, die gegen Fluor austauschbares Halogen enthält, einsetzen. Diese Mengen erweisen sich üblicherweise als ausreichend.

Zu in der Erfindung mit besonderem Erfolg einsetzbaren Verbindungen der Formel III gehören unter anderem
Tetrakis(dimethylamino)phosphoniumchlorid,
Tetrakis(dimethylamino)phosphoniumbromid,
Tetrakis(diethylamino)phosphoniumchlorid,
Tetrakis(diethylamino)phosphoniumbromid,
Tetrakis(dipropylamino)phosphoniumchlorid,
Tetrakis(dipropylamino)phosphoniumbromid,
Tetrakis(dibutylamino)phosphoniumchlorid,
Tetrakis(dibutylamino)phosphoniumbromid,
Tetrakis(pyrrolidino)phosphoniumchlorid,
Tetrakis(pyrrolidino)phosphoniumbromid,
Tetrakis(piperidino)phosphoniumchlorid,
Tetrakis(piperidino)phosphoniumbromid,
Tetrakis(morpholino)phosphoniumchlorid,
Tetrakis(morpholino)phosphoniumbromid,
Tris(dimethylamino)(diethylamino)phosphoniumchlorid,
Tris(dimethylamino)(diethylamino)phosphoniumbromid,
Tris(dimethylamino)(dipropylamino)phosphoniumchlorid,
Tris(dimethylamino)(dipropylamino)phosphoniumbromid,
Tris(dimethylamino)(dibutylamino)phosphoniumchlorid,
Tris(dimethylamino)(dibutyiamino)phosphoniumbromid,
Tris(dimethylamino)(dihexylamino)phosphoniumchlorid,
Tris(dimethylamino)(dihexylamino)phosphoniumbromid,
Tris(dimethylamino)(diheptylamino)phosphoniumchlorid,
Tris(dimethylamino)(diheptylamino)phosphoniumbromid,
Tris(dimethylamino)(cyclopentylamino)phosphoniumchlorid,
Tris(dimethylamino)(cyclopentylamino)phosphoniumbromid,
Tris(dimethylamino)(cyclohexylamino)phosphoniumchlorid,
Tris(dimethylamino)(cyclohexylamino)phosphoniumbromid,
Tris(dimethylamino)(diallylamino)phosphoniumchlorid,
Tris(dimethylamino)(diallylamino)phosphoniumbromid,
Tris(diethylamino)(dimethylamino)phosphoniumchlorid,
Tris(diethylamino)(dimethylamino)phosphoniumbromid,
Tris(diethylamino)(dihexylamino)phosphoniumchlorid,
Tris(diethylamino)(dihexylamino)phosphoniumbromid,
Tris(diethylamino)(diheptylamino)phosphoniumchlorid,
Tris(diethylamino)(diheptylamino)phosphoniumbromid,
Tris(piperidino)(diallylamino)phosphoniumchlorid,
Tris(piperidino)(diallylamino)phosphoniumbromid,
Tris(pyrrolidino)(ethylmethylamino)phosphoniumchlorid,
Tris(pyrrolidino)(ethylmethylamino)phosphoniumbromid,
Tris(pyrrolidino)(diethylamino)phosphoniumchlorid und/oder
Tris(pyrrolidino)(diethylamino)phosphoniumbromid.

Man kann als Katalysator eine Verbindung der Formel III oder ein Gemisch zweier oder mehrerer Verbindungen der Formel III verwenden. Besonders einfach gestaltet sich dies, wenn man Gemische von Verbindungen der Formel III, wie sie bei der Synthese anfallen, verwendet.

Das erfindungsgemäße Verfahren läßt sich in Anwesenheit oder Abwesenheit eines Lösungsmittels durchführen. Werden Lösungsmittel verwendet, so sind sowohl dipolar aprotische und aprotische als auch protische Lösungsmittel geeignet.

Geeignete dipolar aprotische Lösungsmittel sind beispielsweise Dimethylsulfoxid (DMSO), Dimethylsulfon, Sulfolan (TMS), Dimethylformamid (DMFA), Dimethylacetamid, 1,3-Dimethylimidazolin-2-on, N-Methylpyrrolidon, Hexamethylphosphorsäuretriamid, Acetonitril und/oder Benzonitril. Diese Lösungsmittel finden allein oder als Mischung von zwei oder mehreren Anwendung.

Zu geeigneten aprotischen Lösungsmitteln ohne ausgeprägten dipolaren Charakter gehören unter anderem Kohlenwasserstoffe oder chlorierte Kohlenwasserstoffe, beispielsweise Benzol, Toluol, ortho-Xylol, meta-Xylol, para-Xylol, technische Gemische isomerer Xylole, Ethylbenzol, Mesitylen, ortho-Chlortoluol, meta-Chlortoluol, para-Chlortoluol, ortho-Dichlorbenzol, meta-Dichlorbenzol, para-Dichlorbenzol oder Gemische eines oder mehrerer dieser Lösungsmittel.

Das aprotische oder dipolar aprotische Lösungsmittel kann in beliebigen Mengen, beispielsweise 5 bis 500 Gew.-% bezogen auf das Substrat, verwendet werden. Bevorzugt werden allerdings geringe Mengen im Bereich von 5 bis 30 Gew.-%, bezogen auf die Verbindung, die gegen Fluor austauschbares Halogen enthält. Bei der Verwendung von protischen Lösungsmitteln liegen die eingesetzten Mengen im Bereich von 0,1 bis 5, bevorzugt 0,1 bis 2 Gew.-%, bezogen auf das Substrat, das gegen Fluor austauschbares Halogen enthält.

Weiters bevorzugt ist es im Rahmen des erfindungsgemäßen Verfahren, daß die Fluorierung durch Halogenaustauch bei Temperaturen im Bereich von etwa Raumtemperatur bis zur Siedetemperatur des Reaktionsmediums, in vielen Fällen also des Lösungsmittels, oder der Edukte, welche umgesetzt werden sollen, durchgeführt wird, je nachdem, welche Siedetemperatur niedriger ist.

In einer Vielzahl von Fällen genügt es, das erfindungsgemäße Verfahren bei einer Temperatur von 60 bis 250, insbesondere 90 bis 220, bevorzugt 120 bis 200 °C durchzuführen.

Somit hängt die Reaktionstemperatur auch von der Art der Verbindung ab, die gegen Fluor austauschbares Halogen enthält. So erfordern vergleichsweise reaktionsträge Verbindungen in der Regel höhere Reaktionstemperaturen, während vergleichsweise reaktive Ausgangsstoffe sich bereits bei relativ niedrigen Temperaturen erfolgreich umsetzen lassen.

Gleiches trifft auf die Reaktionszeiten zu. Reaktionsträgere Ausgangsstoffe erfordern üblicherweise längere Reaktionszeiten verglichen mit reaktiveren Ausgangsstoffen.

An dieser Stelle sei insbesondere auch noch darauf aufmerksam gemacht, daß ein Austausch lediglich eines Halogenatoms in der Regel einfacher durchzuführen ist, als ein Austausch von zwei oder mehr Halogenatomen gegen Fluor. Der zweifache oder mehrfache Halogen-Fluor-Austausch erfordert, sofern überhaupt möglich, im allgemeinen erheblich schärfere Reaktionsbedingungen (höhere Reaktionstemperaturen und längere Reaktionszeiten) als ein einfacher Halogen-Fluor-Austausch.

Das erfindungsgemäße Verfahren kann sowohl unter reduziertem Druck als auch unter Atmosphärendruck oder Überdruck ausgeübt werden. Diese Möglichkeit wird beispielsweise genutzt, indem geringe Mengen eines leicht siedenden aprotischen Lösungsmittels, das mit Wasser ein Azeotrop bildet, beispielsweise Benzol, Xylol, Mesitylen, Cyclohexan oder Toluol, vor Beginn der Reaktion in die Reaktionssuspension gegeben werden. Anschließend wird ein Teil des Lösungsmittels durch Anlegen eines reduzierten Druckes zusammen mit Wasser aus der Reaktionssuspension wieder entfernt. Durch diese Verfahrensweise läßt sich die Reaktionsgeschwindigkeit und die Ausbeute steigern und darüber hinaus die Bildung von Nebenprodukten minimieren.

Die Verbindung der allgemeinen Formel III kann in Abwesenheit oder Anwesenheit von Luftsauerstoff verwendet werden. Bevorzugt wird das Arbeiten unter Schutzgas, beispielsweise Argon oder Stickstoff.

Bei Durchführung des erfindungsgemäßen Verfahrens ist ferner zu gewährleisten, daß während der gesamten Reaktion das Reaktionsgemisch gut durchmischt wird. Schließlich ist auch die Möglichkeit der kontinuierlichen oder diskontinuierlichen Verfahrensweise bemerkenswert. Bevorzugt ist ein kontinuierlicher Prozeß im technischen Maßstab.

Die Aufarbeitung des Reaktionsgemisches nach der Fluorierung kann wie bereits angedeutet vorteilig durch destillative Auftrennung des Reaktionsgemisches erfolgen und ermöglicht es die Lösemittel zu isolieren und zu recyclieren. Zur wäßrigen Aufarbeitung wird das Gemisch in einen Überschuß Wasser gegossen und die angefallenen Produkte werden abfiltriert oder mit organischen Lösemitteln extrahiert.

Die an sich besonders hohe Effizienz der erfindungsgemäß eingesetzten Verbindungen der Formeln III und IV kann gegebenenfalls durch Zugabe von katalytisch wirkenden Verbindungen weiter verbessert werden. Allgemein lassen sich alle dem Fachmännhierfür z.B. aus den eingangs zitierten Literaturstellen bekannten Katalysatoren einsetzen. Zu den einsetzbaren Katalysatoren gehören unter anderen quaternäre Ammonium-, Phosphonium-, Amidophosphoniumsalze, crown ether, Polyethylenglykole etc.

Besonders vorteilig gestaltet sich das Verfahren der Erfindung bei Zusatz katalytisch wirksamer Mengen von Tetramethylammoniumchlorid, Tetrabutylammoniumchlorid, Tetrabutylphosphoniumbromid, Tetraphenylphosphoniumbromid; Tetrakis(diethylamino)phosphoniumbromid, 18-crown-6, PEG 500 dimethylether.

Die nachfolgenden Beispiele und Vergleichsbeispiele dienen zur Veranschaulichung des Gegenstands der Erfindung, ohne daß die Erfindung auf die Beispiele beschränkt sein soll.

### Beispiel 1

Herstellung von 4-Fluorbenzaldehyd aus 4-Chlorobenzaldehyd.

In einem 500 ml Vierhalskolben, der mit Thermometer, Ankerrührer und Rückflußkühler mit Blasenzähler bestückt ist, werden 140 g (1 mol) 4-Chlorobenzaldehyd, 58 g (1 mol) Kaliumfluorid. 5 g. Nitrobenzol und 7,98 g Tetrakis(diethylamino)phosphoniumbromid (Phasentransfer-Katalysator) vorgelegt. Anschließend erhitzt man unter Rühren auf 190 °C und läßt über 20 h reagieren. Nach Beendigung der Umsetzung läßt man abkühlen, löst das Reaktionsgemisch in Chlorbenzol, filtriert unlösliche Bestandteile ab und reinigt das Produkt (4-Fluorbenzaldehyd) durch fraktianierte Destillation unter reduziertem Druck.
Ausbeute: 77 %
Selektivität: 93 %
Gehalt an Benzaldehyd: 0.01 %

### Vergleichsbeispiel 2

Herstellung von 4-Fluorbenzaldehyd aus 4-Chlorobenzaldehyd.

Man arbeitet wie in Beispiel 1 beschrieben jedoch ohne Nitrobenzol Zusatz.
Ausbeute 75 %
Selektivität 90 %
Gehalt an Benzaldehyd 0,15 %.

### Beispiel 3

Herstellung von 2-Fluorbenzonitril aus 2-Chlorbenzonitril.

In einem 500 ml Vierhalskolben, der mit Thermometer, Ankerrührer und Rückflußkühler mit Blasenzähler bestückt ist, werden 137,5 g (1 mol) 2-Chlorbenzonitril, 58 g (1 mol) Kaliumfluorid, 5 g Nitrobenzol, 7,98 g Tetrakis(diethylamino)phosphoniumbromid (Phasentransfer-Katalysator) und 30 ml Sulfolan vorgelegt. Anschließend erhitzt man unter Rühren auf 190 °C und läßt über 20 h reagieren. Nach Beendigung der Umsetzung läßt man abkühlen, löst das Reaktionsgemisch in Chlorbenzol, filtriert unlösliche Bestandteile ab und reinigt das Produkt (2-Fluorbenzonitril) durch fraktionierte Destillation unter reduziertem Druck.
Ausbeute 94 %
Selektivität 96 %
Gehalt an Benzonitril 0,1%.

### Vergleichsbeispiel 4

Hersteilung von 2-Fluorbenzonitril aus 2-Chlorbenzonitril.

Man arbeitet wie in Beispiel 3 beschrieben jedoch ohne Zusatz von Nitrobenzol.
Ausbeute 92 %
Selektivität 94 %
Gehalt an Benzonitril 0.35 %.

### Beispiel 5

Herstellung von 2-Fluorbenzonitril aus 2-Chlorbenzonitril.

Man arbeitet wie in Beispiel 3 beschrieben, setzt jedoch an Stelle des Nitrobenzols 2,5 g. 4,4.Dinitrodiphenylether ein.
Ausbeute 91 %
Gehalt an Benzonitril 0,01 %.

### Beispiel 6

Herstellung von 2,6-Difluorobenzonitril aus 2,6-Dichlorbezonitril.

In einem 500 ml Vierhalskolben, der mit Thermometer, Ankerrührer und Rückflußkühler mit Blasenzähler bestückt ist, werden 172 g (1 mol) 2,6-Dichlorbezonitril, 116 g (2 mol) Kaliumfluorid, 3 g 4-Fluornitrobenzol, 7,98 g Tetrakis(diethylamino)phosphoniumbromid (Phasentransfer-Katalysator) und 90 ml Sulfolan vorgelegt. Anschließend erhitzt man unter Rühren auf 190 °C und läßt über 15 h reagieren. Nach Beendigung der Umsetzung läßt man abkühlen, löst das Reaktionsgemisch in Chlorbenzol, filtriert unlösliche Bestandteile ab und reinigt das Produkt (2,6-Difluorbenzonitril) durch fraktionierte Destillation unter reduziertem Druck.
Ausbeute 91 %
Selektivität 96 %
Gehalt an 2-Fluorobenzonitril 0,04%.

### Vergleichsbeispiel 7

Herstellung von 2,6-Difluorobenzonitril aus 2,6-Dichlorbenzonitril.

Man arbeitet wie in Beispiel 6 beschrieben jedoch ohne 4-Fluoronitrobenzol.

Gehalt an 2-Fluorobenzonitril: 0,7%.

### Vergleichsbeispiel 8 (gemäß JP 08092148 A2)

Herstellung von 4-Fluorobenzaldehyd aus 4-Chlorbenzaldehyd.

Man arbeitet wie in JP 08092148 A2 beschrieben.
Ausbeute 38,4 %
Gehalt an Benzaldehyd: 0,72%.

### Beispiel 9

Herstellung von 4-Fluorobenzaldehyd aus 4-Chlorbenzaldehyd.

Man arbeitet wie in Vergleichsbeispiel 8, anstelle von TPPB = Tetraphenylphosphoniumbromid nimmt man jedoch TPB = Tetrakis(diethylamino)phosphoniumbromid.
Ausbeute 48 %
Selektivität 85 %
Gehalt an Benzaldehyd: 0,18 %.

### Beispiel 10

Herstellung von 4-Fluorbenzaldehyd aus 4-Chlorobenzaldehyd.

In einem 500 ml Vierhalskolben, der mit Thermometer,. Ankerrührer und Rückflußkühler mit Blasenzähler bestückt ist, werden 140 g (1 mol) 4-Chlorobenzaldehyd, 58 g (1 mol) Kaliumfluorid, 5 g. Dimethylsulfoxid und 7,98 g Tetrakis(diethylamino)phosphoniumbromid (Phasentransfer-Katalysator) vorgelegt. Anschließend erhitzt man unter Rühren auf 190 °C und läßt über 20 h reagieren. Nach Beendigung der Umsetzung läßt man abkühlen, löst das Reaktionsgemisch in Chlorbenzol, filtriert unlösliche Bestandteile ab und reinigt das Produkt (4-Fluorbenzaldehyd) durch fraktionierte Destillation unter reduziertem Druck.
Ausbeute: 74 %
Selektivität: 90 %
Gehalt an Benzaldehyd: 0,013 %

### Beispiel 11

Herstellung von 2-Fluorbenzonitril aus 2-Chlorbenzonitril.

In einem 500 ml Vierhalskolben, der mit Thermometer, Ankerrührer und Rückflußkühler mit Blasenzähler bestückt ist, werden 137,5 g (1 mol) 2-Chlorbenzonitril, 58 g (1 mol) Kaliumfluorid, 5 g Phenylmethylsulfoxid, 7,98 g Tetrakis(diethylamino)phosphoniumbromid (Phasentransfer-Katalysator) und 30 ml Sulfolan vorgelegt. Anschließend erhitzt man unter Rühren auf 190 °C und läßt über 20 h reagieren. Nach Beendigung der Umsetzung läßt man abkühlen, löst das Reaktionsgemisch in Chlorbenzol, filtriert unlösliche Bestandteile ab und reinigt das Produkt (2-Fluorbenzonitril) durch fraktionierte Destillation unter reduziertem Druck.
Ausbeute 90 %
Selektivität 93 %
Gehalt an Benzonitril 0,08 %.

### Beispiel 12

Herstellung von 2,6-Difluorobenzonitril aus 2,6-Dichlorbezonitril.

in einem 500 ml Vierhalskolben, der mit Thermometer, Ankerrührer und Rückflußkühler mit Blasenzähler bestückt ist, werden 172 g (1 mol) 2,6-Dichlorbezonitril, 116 g (2 mol) Kaliumfluorid, 3 g Dimethylsulfoxid, 7,98 g Tetrakis(diethylamino)phosphoniumbromid (Phasentransfer-Katalysator) und 90 ml Sulfolan vorgelegt. Anschließend erhitzt man unter Rühren auf 190 °C und läßt über 15 h reagieren. Nach Beendigung der Umsetzung läßt man abkühlen, löst das Reaktionsgemisch in Chlorbenzol, filtriert unlösliche Bestandteile ab und reinigt das Produkt (2,6-Difluorbenzonitril) durch fraktionierte Destillation unter reduziertem Druck.
Ausbeute 93 %
Selektivität 95 %
Gehalt an 2-Fluorobenzonitril 0,01 %.

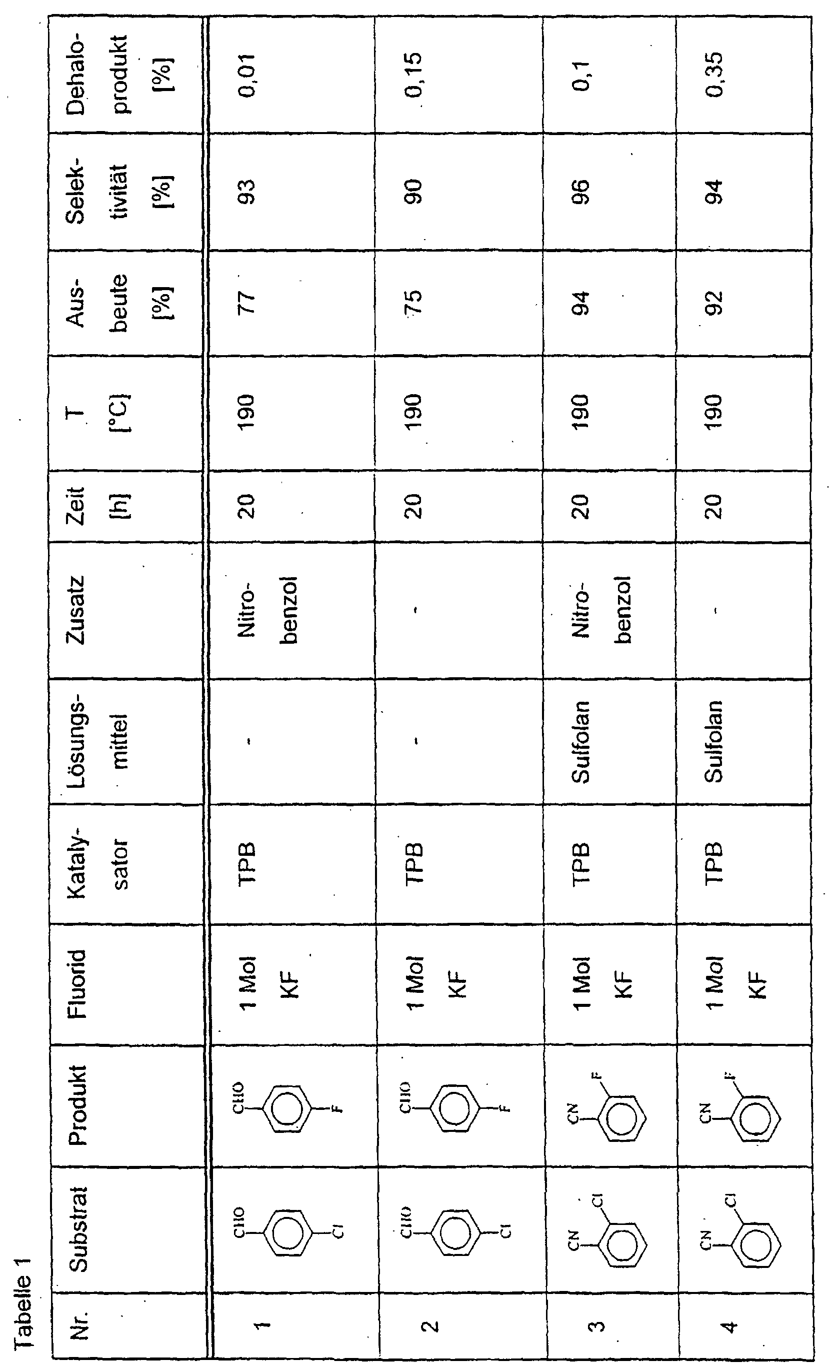

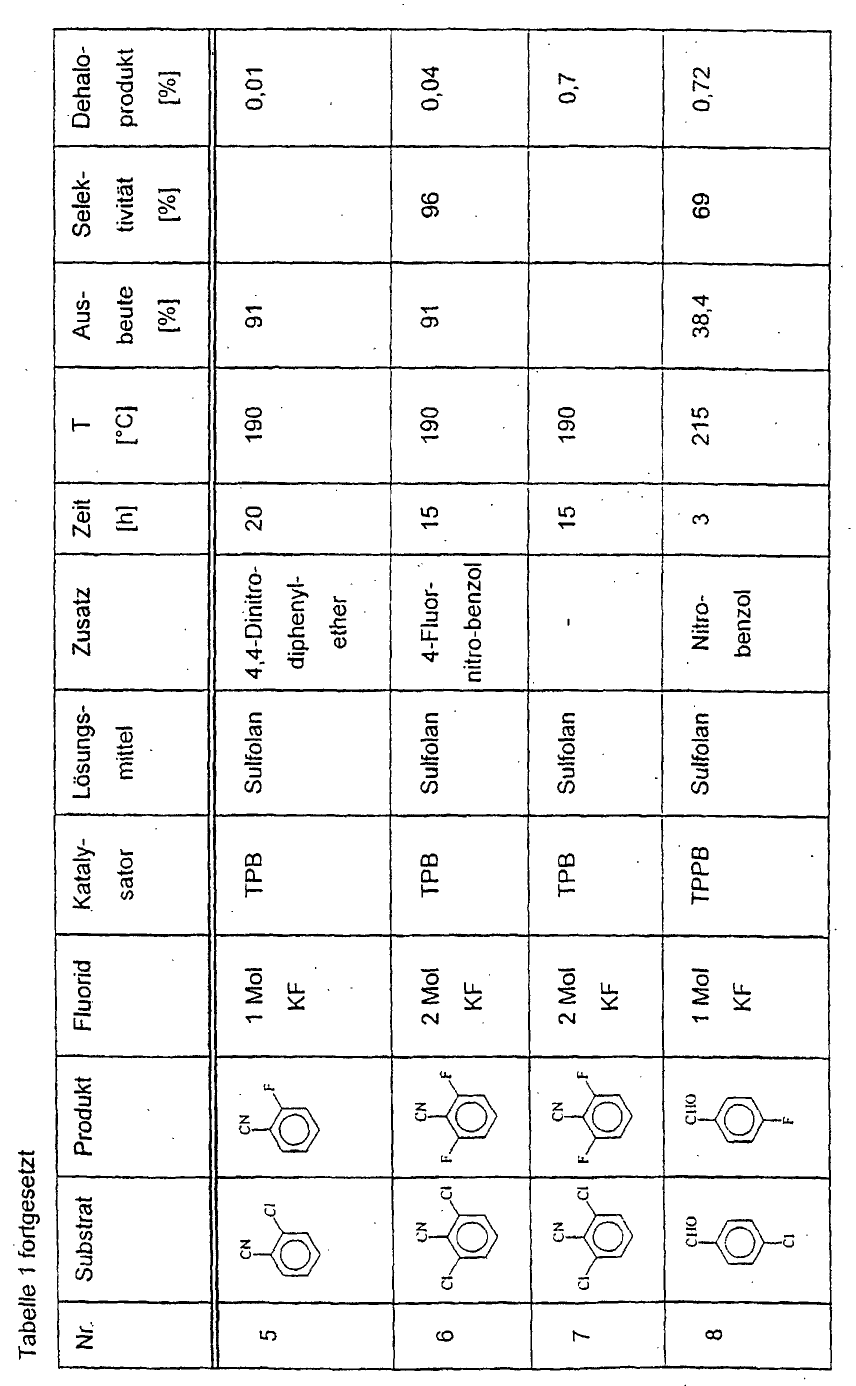

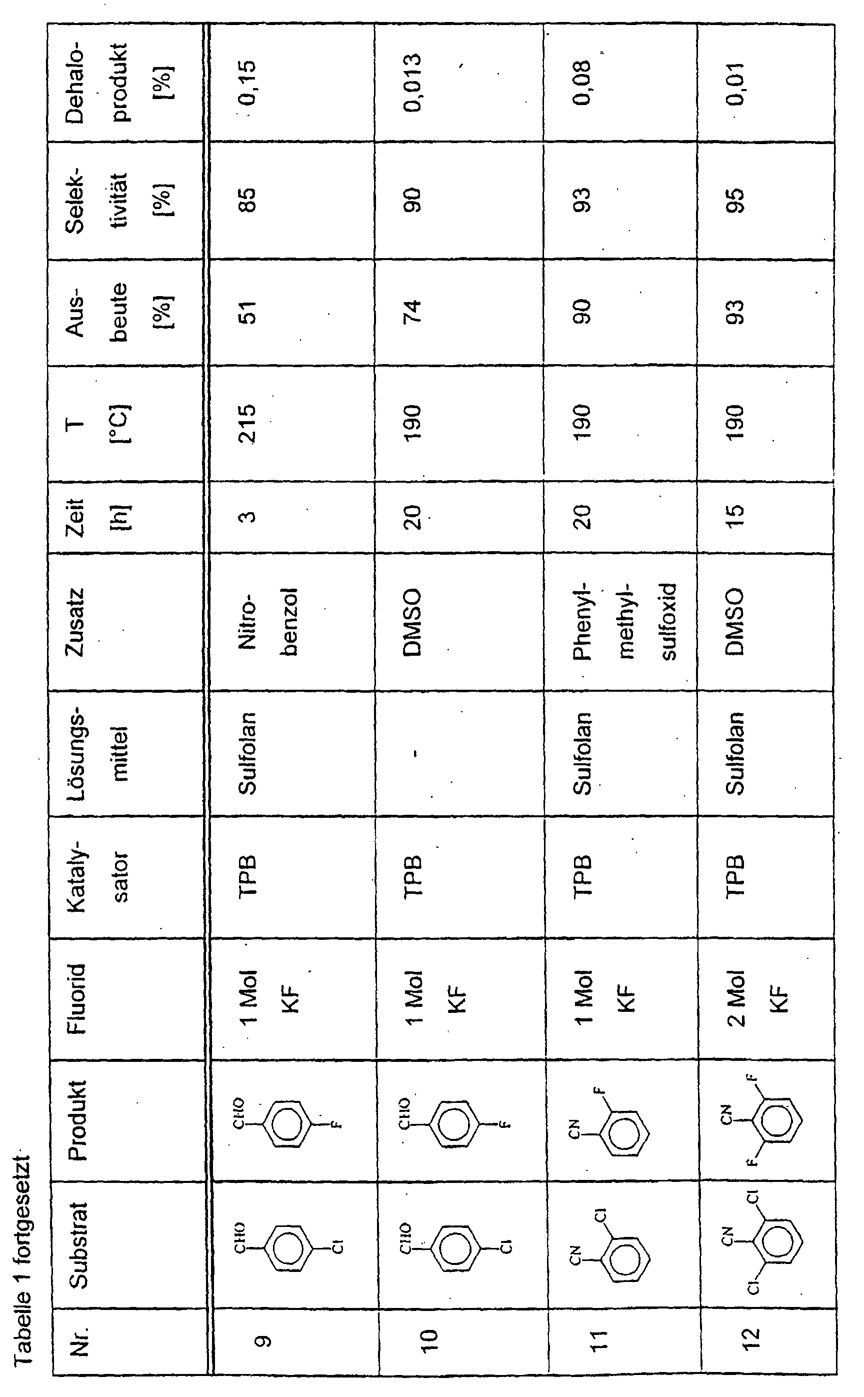

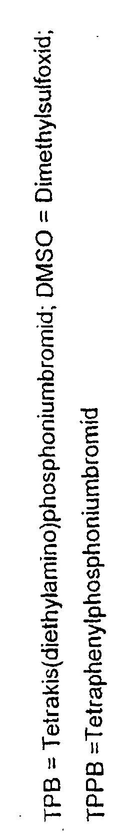

## Patentansprüche

1. Verfahren zur Herstellung von Fluor enthaltenden Verbindungen,**dadurch gekennzeichnet, daß** man eine Verbindung, die eine oder mehrere gegen Fluor austauschbare Halogenatome aufweist, mit einem Fluorid der allgemeinen Formel I oder einem Gemisch von Fluoriden der allgemeinen Formel I
KAT⁺F⁻ (I),
worin KAT⁺ für Alkalimetallion, NH₄⁺, Erdalkalimetallion oder einen Rest der allgemeinen Formel II
A¹A²A³A⁴N⁺ (II),
steht,
wobei A¹, A², A³, A⁴ unabhängig voneinander, gleich oder verschieden sind und geradkettiges oder verzweigtes Alkyl oder Alkenyl mit 1 bis 12 Kohlenstoffatomen, Cycloalkyl mit 4 bis 8 Kohlenstoffatomen, Aryl mit 6 bis 12 Kohlenstoffatomen oder Aralkyl mit 7 bis 12 Kohlenstoffatomen bedeuten, in Anwesenheit einer Verbindung oder eines Gemisches von Verbindungen der allgemeinen Formel III 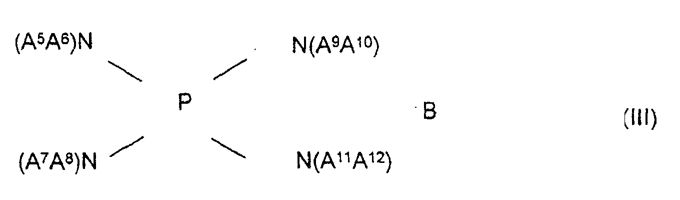 worin A⁵, A⁶, A⁷, A⁸, A⁹, A¹⁰, A¹¹, A¹² unabhängig voneinander, gleich oder verschieden sind und für geradkettiges oder verzweigtes Alkyl oder Alkenyl mit 1 bis 12 Kohlenstoffatomen, Cycloalkyl mit 4 bis 8 Kohlenstoffatomen, für Aryl mit 6 bis 12 Kohlenstorfatomen oder Aralkyl mit 7 bis 12 Kohlenstoffatomen
stehen, oder A⁵A⁶, A⁷A⁸, A⁹A¹⁰, A¹¹A¹² unabhängig voneinander, gleich oder verschieden sind und direkt oder über O oder N-A¹³ miteinander zu einem Ring mit 3 bis 7 Ringgliedern verbunden sind, A¹³ für ein Alkyl mit 1 bis 4 Kohlenstoffatomen steht und B⁻ einen einwertigen Säurerest oder das Äquivalent eines mehrwertigen Säurerestes bedeutet, und in Gegenwart einer oder mehrerer Verbindungen der allgemeinen Formeln IV, umfassend IVa und/oder IVb
X-NO₂ (IVa),
X-SO-X' (IVb),
worin X und X' unabhängig voneinander gleich oder verschieden für (C₈-C₁₈)-Aryl, unsubstituiert oder substituiert, für (C₅-C₁₈)-Aryloxy, substituiert oder unsubstituiert, (C₆-C₁₈)-Arylthio, substituiert oder unsubstituiert, (C₇-C₁₂)-Aralkyl, substituiert oder unsubstituiert, oder einen Rest der Formel V stehen 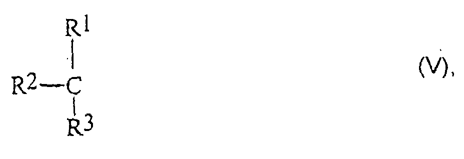 worin R¹, R², R³ unabhängig voneinander, gleich oder verschieden sind und für Wasserstoff, geradkettiges oder verzweigtes Alkyl oder Alkenyl mit 1 bis 12 Kohlenstoffatomen oder Cycloalkyl mit 4 bis 8 Kohlenstoffatomen stehen, in Anwesenheit oder Abwesenheit eines Lösungsmittels bei Temperaturen im Bereich von 40 C bis 260 C umsetzt.

2. Verfahren nach Anspruch 1, **dadurch gekennzeichnet, daß** man die Fluorierung durch Halogenaustausch in Gegenwart von Nitrobenzol, 2-Fluoronitrobenzol, 3-Fluoronitrobenzol, 4-Fluoronitrobenzol, 2,4-Difluoronitrobenzol, 3-Chlornitrobenzol, 2-Nitrotoluol, 3-Nitrotoluol, 4-Nitrotoluol, 2-Nitroanisol, 3-Nitroanisol, 4-Nitroanisol, 2-Nitrothiophen, 4-Nitro-2-propylbenzol, 1-Nitronaphtalin, 2-Nitronaphtalin, 2,4-Dinitrodiphenyl, 4,4'-Dinitrodiphenyl, 4,4'-Dinitrodiphenylether, Dinitrodiphenyldisulfid, Nitromethan, Nitroethan, Nitropropan, Nitroanthracen, 1-Nitropyren, Dimethylsulfoxid, Diphenylsulfoxid, Phenylmethylsulfoxid, Diethylsulfoxid und/oder Methyltrifluoromethylsulfoxid durchführt.

3. Verfahren nach Anspruch 1 oder 2, **dadurch gekennzeichnet, daß** die Fluorierung durch Halogenaustausch in Gegenwart von Nitrobenzol und/oder DMSO durchgeführt wird.

4. Verfahren nach einem oder mehreren der vorhergehenden Ansprüche, **dadurch gekennzeichnet, daß** man die Verbindung der Formeln IVa und/oder IVb in einer Menge von etwa 0,1 bis 20 Gew.-%, in Bezug auf die einzusetzende auszutauschendes Halogen aufweisende Verbindung, einsetzt.

5. Verfahren nach einem oder mehreren der vorhergehenden Ansprüche, **dadurch gekennzeichnet, daß** man die Verbindung der Formeln IVa und/oder IVb in einer Menge von etwa 0,5 bis 10 Gew.-%, vorzugsweise 1 bis 5 Gew.-%, jeweils in Bezug auf die einzusetzende auszutauschendes Halogen aufweisende Verbindung, einsetzt.

6. Verfahren nach einem oder mehreren der vorhergehenden Ansprüche, **dadurch gekennzeichnet, daß** man als Verbindung, die gegen Fluor austauschbares Halogen enthält, eine am Kern wenigstens einen gegen Fluor austauschbaren Chlor- oder Bromsubstituenten besitzende aromatische Verbindung mit 0 bis 3 Stickstoffatome im Kern, die gegebenenfalls einen weiteren, die nucleophiie Substitution begünstigenden Substituenten aufweist, einsetzt.

7. Verfahren nach einem oder mehreren der vorhergehenden Ansprüche, **dadurch gekennzeichnet, daß** man als Ausgangsverbindung, die eine oder mehrere gegen Fluor austauschbare Halogenatomen aufweist, Verbindungen vom Benzol-, Naphthalin-, Pyridin-, Anthracen-, Phenanthren-, Pyrimidin-, Pyrazin-, Chinolin-, Isochinolin-, Acridin-, Acridon-, Cinnolin-, Phthalazin-, Chinazolin-, Chinoxalin-, Phenazin- und/oder Phenoxazin-Typ einsetzt.

8. Verfahren nach einem oder mehreren der vorhergehenden Ansprüche, **dadurch gekennzeichnet, daß** man als Verbindung, die eine oder mehrere gegen Fluor austauschbare Halogenatome aufweist, substituierte Benzaldehyde oder Benzonitrile einsetzt.

9. Verfahren nach einem oder mehreren der vorhergehenden Ansprüche, **dadurch gekennzeichnet, daß** man als Verbindung der allgemeinen Formel I Kaliumfluorid und/oder Cäsiumfluorid einsetzt.

10. Verfahren nach einem oder mehreren der vorhergehenden Ansprüche, **dadurch gekennzeichnet, daß** man als Verbindung der allgemeinen Formel I Tetramethylammoniumfluorid und/oder Tetraphenylammoniumfluorid einsetzt.

11. Verfahren nach einem oder mehreren der vorhergehenden Ansprüche, **dadurch gekennzeichnet, daß** man 0,5 bis 10 Moläquivalente an Verbindung der allgemeinen Formel I auf ein Moläquivalent auszutauschender Halogenatome einsetzt.

12. Verfahren nach einem oder mehreren der vorhergehenden Ansprüche, **dadurch gekennzeichnet, daß** man 1 bis 2 Moläquivalente an Verbindung der allgemeinen Formel I auf ein Moläquivalent auszutauschender Halogenatome einsetzt.

13. Verfahren nach einem oder mehreren der vorhergehenden Ansprüche, **dadurch gekennzeichnet, daß** man die Fluorierung durch Halogenaustausch in Gegenwart einer Verbindung der allgemeinen Formel III durchführt, worin A⁵, A⁶, A⁷, A⁸, A⁹, A¹⁰, A¹¹, A¹², unabhängig voneinander, gleich oder verschieden sind und für geradkettiges oder verzweigtes Alkyl oder Alkenyl mit 1 bis 12 Kohlenstoffatomen oder Cycloalkyl mit 4 bis 8 Kohlenstoffatomen stehen.

14. Verfahren nach einem oder mehreren der vorhergehenden Ansprüche, **dadurch gekennzeichnet, daß** man die Fluorierung durch Halogenaustausch in Gegenwart einer Verbindung der allgemeinen Formel III durchführt, worin A⁵, A⁶, A⁷, A⁸, A⁹, A¹⁰, A¹¹, A¹², unabhängig voneinander, gleich oder verschieden sind und für geradkettiges oder verzweigtes Alkyl oder Alkenyl mit 1 bis.8 Kohlenstoffatomen oder Cycloalkyl mit 5 oder 6 Kohlenstoffatomen stehen.

15. Verfahren nach einem oder mehreren der vorhergehenden Ansprüche, **dadurch gekennzeichnet, daß** man die Fluorierung durch Halogenaustausch in Gegenwart einer Verbindung der allgemeinen Formel III durchführt, worin A⁵, A⁶, A⁷, A⁸, A⁹, A¹⁰, A¹¹, A¹², unabhängig voneinander, gleich oder verschieden sind und für ein geradkettiges oder verzweigtes Alkyl mit 1 bis 4 Kohlenstoffatomen stehen.

16. Verfahren nach einem oder mehreren der vorhergehenden Ansprüche, **dadurch gekennzeichnet, daß** man eine Verbindung der allgemeinen Formel III einsetzt, worin A⁵A⁶ = A⁷A⁸ oder A⁵A⁶ = A⁷A⁸ = A⁹A¹⁰ oder A⁵A⁶ = A⁷A⁸ = A⁹A¹⁰ = A¹¹A¹² ist.

17. Verfahren nach einem oder mehreren der vorhergehenden Ansprüche, **dadurch gekennzeichnet, daß** man eine Verbindung der allgemeinen Formel ill einsetzt, worin A⁵ = A⁶ = A⁷ = A⁸ oder A⁵ = A⁶ = A⁷ = A⁸ = A⁹ = A¹⁰ oder A⁵ = A⁶ = A⁷ = A⁸ = A⁹ = A¹⁰ =A¹¹ = A¹² ist.

18. Verfahren nach einem oder mehreren der vorhergehenden Ansprüche, **dadurch gekennzeichnet, daß** man eine Verbindung der allgemeinen Formel III einsetzt, worin A⁵A⁶ oder A⁵A⁶ und A⁷A⁸ oder A⁵A⁶ und A⁷A⁸ und A⁹A¹⁰ oder A⁵A⁶ und A⁷A⁸ und A⁹A¹⁰ und A¹¹A¹² direkt oder über O oder N-A¹³ miteinander zu einem gesättigten oder ungesättigten Ring mit 5 oder 6 Ringgliedern verbunden sind.

19. Verfahren nach einem oder mehreren der vorhergehenden Ansprüche, **dadurch gekennzeichnet, daß** man eine Verbindung der allgemeinen Formel III einsetzt, worin A⁵A⁶ oder A⁷A⁸ und A⁹A¹⁰ oder A⁵A⁶ und A⁷A⁸ und A⁹A¹⁰ oder A⁵A⁶ und A⁷A⁸ und A⁹A¹⁰ und A¹¹A¹² zu einem Ring, der das N-Atom, an dem die jeweiligen Reste A⁵ bis A¹² sitzen, und gegebenenfalls O oder N-A¹³ und CH₂-Gruppen als Ringglieder umfaßt, verbunden sind.

20. Verfahren nach einem oder mehreren der vorhergehenden Ansprüche, **dadurch gekennzeichnet, daß** man eine Verbindung der allgemeinen Formel III einsetzt, worin B⁻ für F⁻, Cl⁻, Br⁻, J⁻, HF₂⁻, BF₄⁻, C₆H₅SO₃⁻, p-CH₃-C₆H₅SO₃⁻, HSO₄⁻, PF₆⁻ oder CF₃SO₃⁻ steht.

21. Verfahren nach einem oder mehreren der vorhergehenden Ansprüche, **dadurch gekennzeichnet, daß** man eine Verbindung der allgemeinen Formel III einsetzt, worin B⁻ für F⁻, Cl⁻, Br⁻, J⁻, HF₂⁻ oder BF₄⁻ steht.

22. Verfahren nach einem oder mehreren der vorhergehenden Ansprüche, **dadurch gekennzeichnet, daß** man die Verbindung der allgemeinen Formel III in einer Menge von 0,5 bis 35 Gew.-% bezogen auf die Verbindung, die gegen Fluor austauschbares Halogen enthält, einsetzt.

23. Verfahren nach einem oder mehreren der vorhergehenden Ansprüche, **dadurch gekennzeichnet, daß** man als Lösungsmittel ein dipolar aprotisches, ein aprotisches oder ein protisches Lösungsmittel einsetzt.

24. Verfahren nach einem oder mehreren der vorhergehenden Ansprüche, **dadurch gekennzeichnet, daß** man als dipolar aprotisches Lösungsmittel Dimethylsufoxid, Dimethylsulfon, Sulfolan, Dimethylformamid, Dimethylacetamid, 1,3-Dimethylimidazolin-2-on, N-Methylpyrrolidon, Hexamethylphosphorsäuretriamid, Acetonitril, Benzonitril oder ein Gemisch dieser Lösungsmittel einsetzt.

25. Verfahren nach einem oder mehreren der vorhergehenden Ansprüche 1 bis 23, **dadurch gekennzeichnet, daß** man als aprotisches Lösungsmittel einen aromatischen Kohlenwasserstoff, einen chlorierten aromatischen Kohlenwasserstoff oder ein Gemisch dieser Lösungsmittel einsetzt.

26. Verfahren nach Anspruch 25, **dadurch gekennzeichnet, daß** man als aprotisches Lösungsmittel Benzol, Toluol, ortho-Xylol, meta-Xylol, para-Xylol, technische Gemische isomerer Xylole, Ethylbenzol, Mesitylen, ortho-Chlortoluol, meta-Chlortoluol, para-Chlortoluol, ortho-Dichlorbenzol, meta-Dichlorbenzol, para-Dichlorbenzol oder ein Gemisch dieser Lösungsmittel einsetzt.

27. Verfahren nach einem oder mehreren der vorhergehenden Ansprüche, **dadurch gekennzeichnet, daß** man die Umsetzung bei 60 bis 250 °C durchführt.

28. Verfahren nach einem oder mehreren der vorhergehenden Ansprüche, **dadurch gekennzeichnet, daß** man die Umsetzung bei 90 bis 220 °C durchführt.

29. Verfahren nach einem oder mehreren der vorhergehenden Ansprüche, **dadurch gekennzeichnet; daß** man die Umsetzung bei 120 bis 200 °C durchführt.

## Claims

1. A process for preparing fluorine-containing compounds, which comprises reacting a compound containing one or more halogen atoms which can be replaced by fluorine with a fluoride of the formula I or a mixture of fluorides of the formula I
KAT⁺F⁻ (I)
where KAT⁺ is an alkali metal ion, NH₄⁺, an alkaline earth metal ion or a cation of the formula II
A¹A²A³A⁴N⁺ (II)
where A¹, A², A³, A⁴ are, independently of one another, identical or different and are each straight-chain or branched alkyl or alkenyl having from 1 to 12 carbon atoms, cycloalkyl having from 4 to 8 carbon atoms, aryl having from 6 to 12 carbon atoms or aralkyl having from 7 to 12 carbon atoms, in the presence of a compound or a mixture of compounds of the formula III 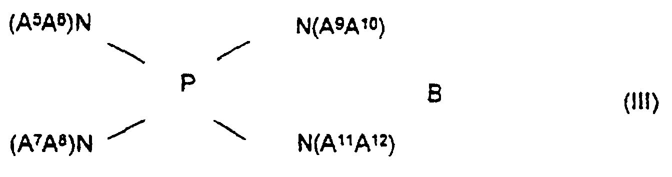 where A⁵, A⁶, A⁷, A⁸, A⁹, A¹⁰, A¹¹, A¹² are, independently of one another, identical or different and are each straight-chain or branched alkyl or alkenyl having from 1 to 12 carbon atoms, cycloalkyl having from 4 to 8 carbon atoms, aryl having from 6 to 12 carbon atoms, aralkyl having from 7 to 12 carbon atoms, or A⁵ A⁶, A⁷ A⁸, A⁹ A¹⁰, A¹¹ A¹² are, independently of one another, identical or different and are bound to one another either directly or via O or N-A¹³ to form a ring having from 3 to 7 ring atoms, A¹³ is alkyl having from 1 to 4 carbon atoms and B⁻ is a monovalent acid anion or the equivalent of a polyvalent acid anion, and in the presence of one or more compounds of the formulae IV encompassing IVa and/or IVb
X-NO₂ (IVa),
X-SO-X' (IVb),
where X and X' are, independently of one another, identical or different and are each substituted or unsubstituted (C₆-C₁₈)-aryl, substituted or unsubstituted (C₆-C₁₈)-aryloxy, substituted or unsubstituted (C₆-C₁₈)-arylthio, substituted or unsubstituted (C₇-C₁₂)-aralkyl or a radical of the formula V 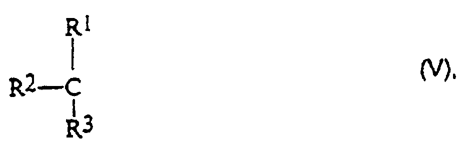 where R¹, R², R³ are, independently of one another, identical or different and are each hydrogen, straight-chain or branched alkyl or alkenyl having from 1 to 12 carbon atoms or cycloalkyl having from 4 to 8 carbon atoms, in the presence or absence of a solvent at temperatures in the range from 40°C to 260°C.

2. The process as claimed in claim 1, wherein the fluorination by halogen exchange is carried out in the presence of nitrobenzene, 2-fluoronitrobenzene, 3-fluoronitrobenzene, 4-fluoronitrobenzene, 2,4-difluoronitrobenzene, 3-chloronitrobenzene, 2-nitrotoluene, 3-nitrotoluene, 4-nitrotoluene, 2-nitroanisole, 3-nitroanisole, 4-nitroanisole, 2-nitrothiophene, 4-nitro-2-propylbenzene, 1-nitronaphthalene, 2-nitronaphthalene, 2,4-dinitrobiphenyl, 4,4'-dinitrobiphenyl, bis(4-nitrophenyl) ether, bis(nitrophenyl) disulfide, nitromethane, nitroethane, nitropropane, nitroanthracene, 1-nitropyrene, dimethyl sulfoxide, diphenyl sulfoxide, phenyl methyl sulfoxide, diethyl sulfoxide and/or methyl trifluoromethyl sulfoxide.

3. The process as claimed in claim 1 or 2, wherein the fluorination by halogen exchange is carried out in the presence of nitrobenzene and/or DMSO.

4. The process as claimed in one or more of the preceding claims, wherein the compound of the formulae IVa and/or IVb is used in an amount of from about 0.1 to 20% by weight, based on the compound containing halogen to be replaced.

5. The process as claimed in one or more of the preceding claims, wherein the compound of the formulae IVa and/or IVb is used in an amount of from about 0.5 to 10% by weight, preferably from 1 to 5% by weight, in each case based on the compound containing halogen to be replaced.

6. The process as claimed in one or more of the preceding claims, wherein the compound containing halogen which can be replaced by fluorine is an aromatic compound which has from 0 to 3 nitrogen atoms in the ring and is substituted on the ring by at least one chlorine or bromine substituent capable of being replaced by fluorine and may be substituted on the ring by a further substituent which promotes nucleophilic substitution of aromatic compounds.

7. The process as claimed in one or more of the preceding claims, wherein the starting compound containing one or more halogen atoms which can be replaced by fluorine is a compound of the benzene, naphthalene, pyridine, anthracene, phenanthrene, pyrimidine, pyrazine, quinoline, isoquinoline, acridine, acridone, cinnoline, phthalazine, quinazoline, quinoxaline, phenazine and/or phenoxazine type.

8. The process as claimed in one or more of the preceding claims, wherein the compound containing one or more halogen atoms which can be replaced by fluorine is a substituted benzaldehyde or benzonitrile.

9. The process as claimed in one or more of the preceding claims, wherein the compound of the formula I which is used is potassium fluoride and/or cesium fluoride.

10. The process as claimed in one or more of the preceding claims, wherein the compound of the formula I which is used is tetramethylammonium fluoride and/or tetraphenylammonium fluoride.

11. The process as claimed in one or more of the preceding claims, wherein from 0.5 to 10 molar equivalents of compound of the formula I are used per molar equivalent of halogen atoms to be replaced.

12. The process as claimed in one or more of the preceding claims, wherein from 1 to 2 molar equivalents of compound of the formula I are used per molar equivalent of halogen atoms to be replaced.

13. The process as claimed in one or more of the preceding claims, wherein the fluorination by halogen exchange is carried out in the presence of a compound of the formula III in which A⁵, A⁶, A⁷, A⁸, A⁹, A¹⁰, A¹¹, A¹² are, independently of one another, identical or different and are each straight-chain or branched alkyl or alkenyl having from 1 to 12 carbon atoms or cycloalkyl having from 4 to 8 carbon atoms.

14. The process as claimed in one or more of the preceding claims, wherein the fluorination by halogen exchange is carried out in the presence of a compound of the formula III in which A⁵, A⁶, A⁷, A⁸, A⁹, A¹⁰, A¹¹, A¹² are, independently of one another, identical or different and are each straight-chain or branched alkyl or alkenyl having from 1 to 8 carbon atoms or cycloalkyl having 5 or 6 carbon atoms.

15. The process as claimed in one or more of the preceding claims, wherein the fluorination by halogen exchange is carried out in the presence of a compound of the formula III in which A⁵, A⁶, A⁷, A⁸, A⁹, A¹⁰, A¹¹, A¹² are, independently of one another, identical or different and are each straight-chain or branched alkyl having from 1 to 4 carbon atoms.

16. The process as claimed in one or more of the preceding claims, wherein use is made of a compound of the formula III in which A⁵A⁶ = A⁷A⁸ or A⁵A⁶ = A⁷A⁸ = A⁹A¹⁰ or A⁵A⁶ = A⁷A⁸ = A⁹A¹⁰ = A¹¹A¹².

17. The process as claimed in one or more of the preceding claims, wherein use is made of a compound of the formula III in which A⁵ = A⁶ = A⁷ = A⁸ or A⁵ = A⁶ = A⁷ = A⁸ = A⁹ = A¹⁰ or A⁵ = A⁶ = A⁷ = A⁸ = A⁹ = A¹⁰ = A¹¹ = A¹².

18. The process as claimed in one or more of the preceding claims, wherein use is made of a compound of the formula III in which A⁵A⁶ or A⁵A⁶ and A⁷A⁸ or A⁵A⁶ and A⁷A⁸ and A⁹A¹⁰ or A⁵A⁶ and A⁷A⁸ and A⁹A¹⁰ and A¹¹A¹² are joined to one another either directly or via O or N-A¹³ to form a saturated or unsaturated ring having 5 or 6 ring atoms.

19. The process as claimed in one or more of the preceding claims, wherein use is made of a compound of the formula III in which A⁵A⁶ or A⁷A⁸ and A⁹A¹⁰ or A⁵A⁶ and A⁷A⁸ and A⁹A¹⁰ or A⁵A⁶ and A⁷A⁸ and A⁹A¹⁰ and A¹¹A¹² are joined to form a ring including, as ring members, the N atom on which the respective radicals A⁵ to A¹² are located and possibly O or N-A¹³ and CH₂ groups.

20. The process as claimed in one or more of the preceding claims, wherein use is made of a compound of the formula III in which B⁻ is F⁻, Cl⁻, Br⁻, I⁻, HF₂⁻, BF₄⁻, C₆H₅SO₃⁻, P-CH₃-C₆H₅SO₃⁻, HSO₄⁻. PF₆⁻ or CF₃SO₃⁻.

21. The process as claimed in one or more of the preceding claims, wherein use is made of a compound of the formula III in which B⁻ is F⁻, Cl⁻, Br⁻, I⁻, HF₂⁻ or BF₄⁻.

22. The process as claimed in one or more of the preceding claims, wherein the compound of the formula III is used in an amount of from 0.5 to 35% by weight, based on the compound containing halogen which can be replaced by fluorine.

23. The process as claimed in one or more of the preceding claims, wherein a dipolar aprotic, an aprotic or a protic solvent is used.

24. The process as claimed in one or more of the preceding claims, wherein dimethyl sulfoxide, dimethyl sulfone, sulfolane, dimethylformamide, dimethylacetamide, 1,3-dimethylimidazolin-2-one, N-methylpyrrolidone, hexamethylphosphoramide, acetonitrile, benzonitrile or a mixture of these is used as dipolar aprotic solvent.

25. The process as claimed in one or more of the preceding claims 1 to 23, wherein an aromatic hydrocarbon, a chlorinated aromatic hydrocarbon or a mixture of these is used as aprotic solvent.

26. The process as claimed in claim 25, wherein benzene, toluene, ortho-xylene, meta-xylene, paraxylene, an industrial mixture of isomeric xylenes, ethylbenzene, mesitylene, ortho-chlorotoluene, meta-chlorotoluene, para-chlorotoluene, orthodichlorobenzene, meta-dichlorobenzene, paradichlorobenzene or a mixture of these is used as aprotic solvent.

27. The process as claimed in one or more of the preceding claims, wherein the reaction is carried out at from 60 to 250°C.

28. The process as claimed in one or more of the preceding claims, wherein the reaction is carried out at from 90 to 220°C.

29. The process as claimed in one or more of the preceding claims, wherein the reaction is carried out at from 120 to 200°C.

## Revendications

1. Procédé pour la préparation de composés contenant du fluor, **caractérisé en ce qu'**on fait réagir un composé qui présente un ou plusieurs atomes d'halogènes échangeables contre du fluor, sur un fluorure de formule générale I ou un mélange de fluorures de formule générale I
KAT⁺F⁻ (I),
où KAT⁺ représente un ion métal alcalin, NH₄⁺, un ion métal alcalino-terreux ou un reste de formule générale (II)
A¹A²A³A⁴N⁺ (II),
où A¹, A², A³, A⁴ sont indépendamment les uns des autres identiques ou différents et représentent des groupes alkyle ou alcényle à chaîne droite ou ramifiée présentant 1 à 12 atomes de carbone, cycloalkyle présentant 4 à 8 atomes de carbone, aryle présentant 6 à 12 atomes de carbone ou aralkyle présentant 7 à 12 atomes de carbone, en présence d'un composé ou d'un mélange de composés de formule générale III 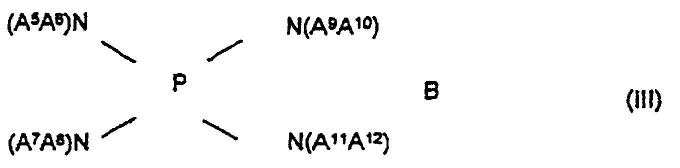 où A⁵, A⁶, A⁷, A⁸, A⁹, A¹⁰, A¹¹, A¹² sont indépendamment les uns des autres identiques ou différents et représentent des groupes alkyle ou alcényle à chaîne droite ou ramifiée présentant 1 à 12 atomes de carbone, cycloalkyle présentant 4 à 8 atomes de carbone, aryle présentant 6 à 12 atomes de carbone ou aralkyle présentant 7 à 12 atomes de carbone, ou A⁵A⁶, A⁷A⁸, A⁹A¹⁰, A¹¹A¹² sont indépendamment les uns des autres identiques ou différents et sont reliés les uns aux autres directement ou par l'intermédiaire de groupes O ou N-A¹³ en un cycle de 3 à 7 chaînons, A¹³ représente un groupe alkyle présentant 1 à 4 atomes de carbone et B⁻ représente un reste acide monovalent ou l'équivalent d'un reste acide multivalent et en présence d'un ou plusieurs composés de formules générales IV, comprenant IVa et/ou IVb
X-NO₂ (IVa),
X-SO-X' (IVb),
où X et X' sont indépendamment l'un de l'autre identiques ou différents et représentent un groupe aryle en C₆-C₁₈ non substitué ou substitué, un groupe (aryl en C₆-C₁₈)oxy substitué ou non substitué, un groupe (aryl en C₆-C₁₈)thio substitué ou non substitué, un groupe arylalkyle en C₇-C₁₂ substitué ou non substitué, ou un reste de formule V 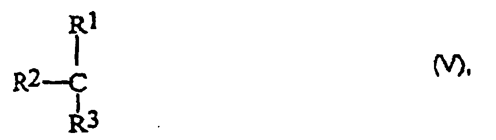 où R¹, R², R³ sont indépendamment les uns des autres identiques ou différents et représentent des atomes d'hydrogène, des groupes alkyle ou alcényle à chaîne droite ou ramifiée présentant 1 à 12 atomes de carbone ou cycloalkyle présentant 4 à 8 atomes de carbone, en présence ou en l'absence d'un solvant à des températures dans le domaine de 40°C à 260°C.

2. Procédé selon la revendications 1, **caractérisé en ce qu'**on met en oeuvre la fluoration par échange d'halogènes en présence de nitrobenzène, de 2-fluoronitrobenzène, de 3-fluoronitrobenzène, de 4-fluoronitrobenzène, de 2,4-difluoronitrobenzène, de 3-chloronitrobenzène, de 2-nitrotoluène, de 3-nitrotoluène, de 4-nitrotoluène, de 2-nitroanisole, de 3-nitroanisole, du 4-nitroanisole, du 2-nitrothiophène, de 4-nitro-2-propylbenzène, de 1-nitronaphtalène, de 2-nitronaphtalène, de 2,4-dinitrophényle, de 4,4'-dinitrophényle, d'éther 4,4'-dinitrodiphénylique, de disulfure de dinitrodiphényle, de nitrométhane, de nitroéthane, de nitropropane, de nitroanthracène, de 1-nitropyrène, de diméthylsulfoxyde, de diphénylsulfoxyde, de phénylméthylsulfoxyde, de diéthylsulfoxyde et/ou de méthyltrifuorométhylsulfoxyde.

3. Procédé selon la revendication 1 ou 2, **caractérisé en ce qu'**on met en oeuvre la fluoration par échange d'halogène en présence de nitrobenzène et/ou DMSO.

4. Procédé selon une ou plusieurs des revendications précédentes, **caractérisé en ce qu'**on utilise le composé de formules IVa et/ou IVb dans une quantité d'environ 0,1 à 20 % en poids, par rapport au composé à utiliser contenant l'atome d'halogène à échanger.

5. Procédé selon une ou plusieurs des revendications précédentes, **caractérisé en ce qu'**on utilise le composé de formules IVa et/ou IVb dans une quantité d'environ 0,5 à 10 % en poids, de préférence de 1 à 5 % en poids chaque fois par rapport au composé à utiliser contenant l'atome d'halogène à échanger.

6. Procédé selon une ou plusieurs des revendications précédentes, **caractérisé en ce qu'**on utilise en tant que composé contenant un atome d'halogène à échanger contre l'atome de fluor, un composé aromatique ayant 0 à 3 atiomes d'azote dans le noyau, présentant sur le noyau au moins un substituants chloro ou bromo échangeable contre du fluor, qui présente éventuellement un autre substituant favorisant la substitution nucléophile.

7. Procédé selon une ou plusieurs des revendications précédentes, **caractérisé en ce qu'**on utilise, en tant que composé de départ qui présente un ou plusieurs atomes d'halogènes échangeables contre du fluor, des composés de type benzène, naphtalène, pyridine, anthracène, phénanthrène, pyrimidine, pyrazine, quinoléine, isoquinoléine, acridine, acridone, cinnoline, phtalazine, quinazoline, quinoxaline, phénazine et/ou phénoxazine.

8. Procédé selon une ou plusieurs des revendications précédentes, **caractérisé en ce qu'**on utilise le benzaldéhyde ou le benzonitrile substitués en tant que composé qui présente un ou plusieurs atomes d'halogènes échangeables contre du fluor.

9. Procédé selon une ou plusieurs des revendications précédentes, **caractérisé en ce qu'**on utilise, en tant que composé de formule générale I, le fluorure de potassium et/ou le fluorure de césium.

10. Procédé selon une ou plusieurs des revendications précédentes, **caractérisé en ce qu'**on utilise, en tant que composé de formule générale I, le fluorure de tétraméthylammonium et/ou le fluorure de tétraphénylammonium.

11. Procédé selon une ou plusieurs des revendications précédentes, **caractérisé en ce qu'**on utilise 0,5 à 10 équivalents molaires de composé de formule générale I pour 1 équivalent molaire d'atomes d'halogène à échanger.

12. Procédé selon une ou plusieurs des revendications précédentes, **caractérisé en ce qu'**on utilise 1 à 2 équivalents molaires de composé de formule générale I pour un équivalent molaire d'atomes d'halogène à échanger.

13. Procédé selon une ou plusieurs des revendications précédentes, **caractérisé en ce qu'**on met en oeuvre la fluoration par échange d'halogène en présence d'un composé de formule générale III, où A⁵, A⁶, A⁷, A⁸, A⁹, A¹⁰, A¹¹, A¹² sont indépendamment les uns des autres identiques ou différents et représentent des groupes alkyle ou alcényle à chaîne droite ou ramifiée présentant 1 à 12 atomes de carbone ou cycloalkyle présentant 4 à 8 atomes de carbone.

14. Procédé selon une ou plusieurs des revendications précédentes, **caractérisé en ce qu'**on met en oeuvre la fluoration par échange d'halogène en présence d'un composé de formule générale III, où A⁵, A⁶, A⁷, A⁸, A⁹, A¹⁰, A¹¹, A¹² sont indépendamment les uns des autres identiques ou différents et représentent des groupes alkyle ou alcényle à chaîne droite ou ramifiée présentant 1 à 8 atomes de carbone, cycloalkyle présentant 5 à 6 atomes de carbone.

15. Procédé selon une ou plusieurs des revendications précédentes, **caractérisé en ce qu'**on met en oeuvre la fluoration par échange d'halogène en présence d'un composé de formule générale III, où A⁵, A⁶, A⁷, A⁸, A⁹, A¹⁰, A¹¹, A¹² sont indépendamment les uns des autres identiques ou différents et représentent un groupe alkyle à chaîne droite ou ramifiée présentant 1 à 4 atomes de carbone.

16. Procédé selon une ou plusieurs des revendications précédentes, **caractérisé en ce qu'**on utilise un composé de formule générale III, où A⁵A⁶ = A⁷A⁸ ou A⁵A⁶ = A⁷A⁸ = A⁹A¹⁰ ou A⁵A⁶ = A⁷A⁸ = A⁹A¹⁰ = A¹¹A¹².

17. Procédé selon une ou plusieurs des revendications précédentes, **caractérisé en ce qu'**on utilise un composé de formule générale III, où A⁵ = A⁶ = A⁷ = A⁸ ou A⁵ = A⁶ = A⁷ = A⁸ = A⁹ = A¹⁰ ou A⁵ = A⁶ = A⁷ = A⁸ = A⁹ = A¹⁰ = A¹¹ = A¹².

18. Procédé selon une ou plusieurs des revendications précédentes, **caractérisé en ce qu'**on utilise un composé de formule générale III, où A⁵A⁶ ou A⁵A⁶ et A⁷A⁸ ou A⁵A⁶ et A⁷A⁸ et A⁹A¹⁰ ou A⁵A⁶ et A⁷A⁸ et A⁹A¹⁰ et A¹¹A¹² sont reliés les uns aux autres directement ou par l'intermédiaire de groupes O ou N-A¹³ en un cycle saturé ou insaturé à 5 ou 6 chaînons.

19. Procédé selon une ou plusieurs des revendications précédentes, **caractérisé en ce qu'**on utilise un composé de formule générale III, où A⁵A⁶ ou A⁷A⁸ et A⁹A¹⁰ ou A⁵A⁶ et A⁷A⁸ et A⁹A¹⁰ ou A⁵A⁶ et A⁷A⁸ et A⁹A¹⁰ et A¹¹A¹² sont reliés les uns aux autres en un cycle qui comprend l'atome de N, portant les restes A⁵ à A¹² respectifs et qui comprend éventuellement des groupes O ou N-A¹³ et CH₂ en tant que chaînons de cycle.

20. Procédé selon une ou plusieurs des revendications précédentes, **caractérisé en ce qu'**on utilise un composé de formule générale III, où B⁻ représente F⁻, Cl⁻, Br⁻, J⁻, HF₂⁻, BF₄⁻, C₆H₅SO₃⁻, p-CH₃-C₆H₅SO₃⁻, HSO₄⁻, PF₆⁻ ou CF₃SO₃⁻.

21. Procédé selon une ou plusieurs des revendications précédentes, **caractérisé en ce qu'**on utilise un composé de formule générale III, où B⁻ représente F⁻, Cl⁻, Br⁻, J⁻, HF₂⁻ ou BF₄⁻.

22. Procédé selon une ou plusieurs des revendications précédentes, **caractérisé en ce qu'**on utilise le composé de formule générale III dans une quantité de 0,5 à 35 % en poids, par rapport au composé présentant l'atome d'halogène à échanger à utiliser.

23. Procédé selon une ou plusieurs des revendications précédentes, **caractérisé en ce qu'**on utilise en tant que solvant un solvant dipolaire aprotique, un solvant aprotique ou protique.

24. Procédé selon une ou plusieurs des revendications précédentes, **caractérisé en ce qu'**on utilise en tant que solvant un solvant dipolaire aprotique le diméthylsulfoxyde, la diméthylsulfone, le sulfolane, le diméthylformamide, le diméthylacétamide, la 1,3-diméthylimidazolin-2-one, la N-méthylpyrrolidone, le triamide de l'acide hexaméthylphosphorique, l'acétonitrile, le benzonitrile ou un mélange de ces solvants.

25. Procédé selon une ou plusieurs des revendications précédentes 1 à 23, **caractérisé en ce qu'**on utilise en tant que solvant aprotique un hydrocarbure aromatique, un hydrocarbure aromatique chloré ou un mélange de ces solvants.

26. Procédé selon la revendication 25, **caractérisé en ce qu'**on utilise en tant que solvant aprotique le benzène, le toluène, l'ortho-xylène, le méta-xylène, le para-xylène, des mélanges techniques d'isomères de xylène, l'éthylbenzène, le mésitylène, l'ortho-chlorotoluène, le méta-chlorotoluène, le parachlorotoluène, l'ortho-dichlorobenzène, le métadichlorobenzène, le para-dichlorobenzène ou un mélange de ces solvants.

27. Procédé selon une ou plusieurs des revendications précédentes, **caractérisé en ce qu'**on met en oeuvre la réaction à une température de 60 à 250°C.

28. Procédé selon une ou plusieurs des revendications précédentes, **caractérisé en ce qu'**on met en oeuvre la réaction à une température de 90 à 220°C.

29. Procédé selon une ou plusieurs des revendications précédentes, **caractérisé en ce qu'**on met en oeuvre la réaction à une température de 120 à 200°C.
